# EUROPÄISCHE PATENTSCHRIFT

(11) **EP 0 815 110 B1**
(45) Veröffentlichungstag und Bekanntmachung des Hinweises auf die Patenterteilung: **28.04.1999**
(21) Anmeldenummer: 95935329.3
(22) Anmeldetag: 10.10.1995
(51) Int. Cl.: C07D 471/00, A61K 31/435, C07D 471/04

(54) **HALOGENIERTE -CARBOLIN-DERIVATE, VERFAHREN ZU IHRER HERSTELLUNG UND VERWENDUNG DIESER SUBSTANZEN ZUR HEMMUNG DER ATMUNGSKETTE**
HALOGENATED -CARBOLINE DERIVATIVES, PROCESS FOR THEIR PREPARATION AND USE OF THESE SUBSTANCES FOR INHIBITING THE RESPIRATORY CHAIN
DERIVES HALOGENES DE -CARBOLINES, LEUR PROCEDE DE PRODUCTION ET UTILISATION DE CES SUBSTANCES POUR L'INHIBITION DE LA CHAINE RESPIRATOIRE

(30) Priorität: 10.10.1994 DE 4436190
(43) Veröffentlichungstag der Anmeldung: 07.01.1998
(73) Patentinhaber: Bringmann, Gerhard, 97074 Würzburg (DE); Reichmann, Heinz, Neurologische Klinik und Poliklinik der Universität Würzburg, 97080 Würzburg (DE); Feineis, Doris, 97074 Würzburg (DE); God, Ralf, 97074 Würzburg (DE); Janetzky, Bernd, Neurologische Klinik und Poliklinik der Universität Würzburg, 97080 Würzburg (DE)
(72) Erfinder: Bringmann, Gerhard, 97074 Würzburg (DE); Reichmann, Heinz, Neurologische Klinik und Poliklinik der Universität Würzburg, 97080 Würzburg (DE); Feineis, Doris, 97074 Würzburg (DE); God, Ralf, 97074 Würzburg (DE); Janetzky, Bernd, Neurologische Klinik und Poliklinik der Universität Würzburg, 97080 Würzburg (DE)
(74) Vertreter: Schüssler, Andrea, Dr.
(86) Internationale Anmeldenummer: DE9501403
(87) Internationale Veröffentlichungsnummer: WO9611197

(56) Entgegenhaltungen:
- GB-A- 975 835
- BULLETIN DE LA SOCIETE CHIMIQUE DE FRANCE 2 PARTIE - CHIMIE ORGANIQUE, BIOCHIMIE., Nr. 4, 1973 PARIS FR, Seiten 1424-1426, M. JULIA 'Sur une nouvelle voie d'accès aux tryptamines'
- LIEBIGS ANNALEN DER CHEMIE, 1991 WEINHEIM DE, Seiten 1189-1194, G. BRINGMANN ET AL. 'Potential tryptophan-derived alkaloids in chloral-treated patients: synthesisnand sterepstructure' in der Anmeldung erwähnt
- JOURNAL OF MEDICINAL CHEMISTRY, Bd. 34, 1991 WASHINGTON US, Seiten 2624-2633, V. VECCHIETTI ET AL. '(1S)-1-(Aminomethyl)-2-(arylacetyl)-1,2,3 ,4-tetrahydroquinoline and heterocycle-condensed tetrahydropyridine derivatives: members of a novel class of very potent k opioid analgesics'
- CHEMICAL ABSTRACTS, vol. 114, no. 5, 1991 Columbus, Ohio, US; abstract no. 42368d, G. BRINGMANN 'Endogenous alkaloids in man. VII. 1-(Trichloromethyl)-1,2,3,4-tetrahydro-bet a-carboline. Apotential chloral-derived indole alkaloid in man' Seite 706; & ARCH. PHARM. (WEINHEIM, GER.), Bd. 323, Nr. 9, 1990 Seiten 567-9,
- JOURNAL OF FLUORINE CHEMISTRY, Bd. 43, 1989 LAUSANNE CH, Seiten 189-206, YASUO MAKI ET AL. 'Synthesis of 1-trifluoromethyl-beta-carboline derivatives'
- CHEMICAL ABSTRACTS, vol. 106, no. 3, 1987 Columbus, Ohio, US; abstract no. 18907t, S. SIDDIQUI ET AL. 'Reaction of harmidine/harmaline with cyanogen bromide. A facile mono- and dibromination at the alpha-carbon of the imine tautomer' Seite 638; & Z. NATURFORSCH., B: ANORG. CHEM., ORG. CHEM., Bd. 40b, Nr. 12, 1985 Seiten 1747-8,
- CHEMICAL ABSTRACTS, vol. 110, no. 1, 1989 Columbus, Ohio, US; abstract no. 2895j, Y. MAKI ET AL. '1-Trifluoromethylcarbolines, their preparation, and their use as plant disease-controlling agents' Seite 270; & JP,A,63 179 874 (SUMITOMO)

## Beschreibung

Diese Erfindung betrifft halogenierte β-Carbolin-Derivate, Verfahren zu ihrer Herstellung und die Verwendung dieser Substanzen zur Hemmung der Atmungskette.

Das bei der Glycolyse, der Fettsäureoxydation und dem Citratzyklus entstehende NADH stellt ein sehr energiereiches Molekül dar, das ein Elektronenpaar mit hohem Übertragungspotential besitzt. Bei der Übertragung dieser Elektronen auf molekularen Sauerstoff wird sehr viel Energie frei, die bei der oxidativen Phosphorylierung zur Erzeugung von ATP, der eigentlichen Energie-"währung" der Zelle, genutzt wird. Dem Komplex I der mitochondrialen Atmungskette, der NADH-CoQ-Reduktase, kommt daher bei der oxidativen Phosphorylierung eine zentrale Stellung zu. Er überträgt in einem ersten Schritt die bei der Oxidation von NADH freiwerdenden Elektronen auf das Coenzym Q. In einem geringeren stöchiometrischen Umfang werden noch über ein weiteres, jedoch energieärmeres Energieäquivalent, FADH₂, Elektronen mittels des Komplex II (Succinat-Ooenzym-Q-Reduktase) auf das Coenzym Q übertragen. Im Gegensatz zur NADH-Oxidation trägt jedoch die FADH₂-Oxidation nur in geringem Umfang zur Energieversorgung der Zelle bei. Wird daher insbesondere Komplex I gehemmt, hat es für Gewebe mit hohem Energieverbrauch bzw. aerobem Stoffwechsel drastische Auswirkungen, die in der Regel zum Absterben der betroffenen Zellen bzw. Gewebe führen. Bei verschiedenen Krankheiten kommt es zum Absterben von Zellen, indem die Atmungskette durch verschiedene Stoffwechselprozesse oder durch Medikamenten-Nebenwirkungen gehemmt wird. Aus diesem Grund besteht ein Bedarf für Hemmstoffe der Atmungskette, um diese *in vivo* oder in Zellkultur auf Zellen wirken zu lassen und dabei die Zusammenhänge des Absterbens von Zellen aufzuklären. Dazu können kultivierten Zellen oder Tieren die Hemmstoffe verabreicht werden und an den gehemmten Organismen könnten z.B. Medikamente gegen Krankheiten ausgetestet werden, bei denen eine gestörte Atmungskette eine Rolle spielt. Diese Hemmstoffe sind somit ein Hilfsmittel entweder um Störungen im mitochondrialen Energiestoffwechsel zu erforschen oder um auszutesten, ob bestimmte Medikamente der Störung der Atmungskette entgegenwirken.

Die Aufgabe der vorliegenden Erfindung bestand deshalb darin, Hemmstoffe für die Atmungskette, insbesondere für die Komplexe I und/oder II, bereitzustellen.

Diese Aufgabe wird überraschenderweise durch halogenierte β-Carbolin-Derivate gelöst. Diese β-Carbolin-Derivate haben die allgemeine Hauptgrundstruktur (**I**):

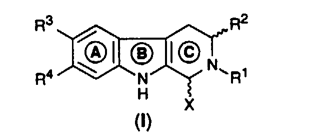

Im Ring C können dabei eine, zwei oder drei Doppelbindungen vorhanden sein.

In der Formel (**I**) kann X -C(Hal)₃, -CH(Hal)₂, -CH₂(Hal) oder eine =C(Hal)₂-Gruppe mit Hal = F, Cl, Br, I sein.

Die Substituenten R¹, R², R³ und R⁴ haben dabei folgende Bedeutungen:
R¹ kann -H, eine C₁-C₁₈ Alkylgruppe (z.B. Methyl, Ethyl, Propyl, *n*-Butyl, *iso*-Butyl, *tert.*-Butyl usw.), -CH₂-CH=CH₂, -CH₂-C₆H₅, eine Acylgruppe (z.B. Formyl, Acetyl, Monochloracetyl, Trichloracetyl usw.) oder Aroylgruppe sein, mit der Maßgabe, daß R¹ auch ein freies Elektronenpaar sein kann, wenn der Stickstoff an der Ausbildung einer Doppelbindung beteiligt ist,
R² ist -H, -COOH, -COOCH₃, -COOC₂H₅, -COOCH₂-CH=CH₂, -COOCH₂- C₆H₅, -COONH₂, -CO₂NR₂, CH₂OAc oder -CH₂-OH,
R³ ist H-, HO-, CH₃O-, C₂H₅O-, C₆H₅-CH₂O-, CH₃COO- oder C₂H₅COO-.
R⁴ ist H-, HO-, CH₃O-, C₂H₅O-, C₆H₅-CH₂O-, CH₃COO- oder C₂H₅COO-,

Es ergeben sich bevorzugte Untergrundstrukturen: 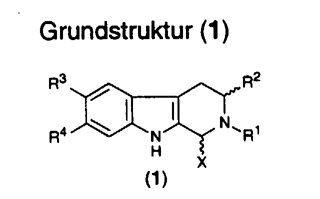 bevorzugt
X = -CCl₃, R¹ = -C₂H₅, R²-R⁴ = -H
X = -CCl₃, R¹ = -COCH₂Cl, R²-R⁴ = -H
X = -CCl₃, R¹ = -COCCl₃, R²-R⁴ = -H
X = -CCl₃, R¹-R² = -H, R³ = -OH, R⁴ = -H
X = -CCl₃, R¹-R² = -H, R³ = -OCH₃, R⁴ = -H
X = -CCl₃, R¹-R² = -H, R³ = -H, R⁴ = -OH
X = -CCl₃, R¹-R² = -H, R³ = -H, R⁴ = -OCH₃
X = -CBr₃, R¹-R⁴ = -H
X = -CBr₃, R¹ = -CHO, R²-R⁴ = -H
ganz bevorzugt
X = -CCl₃, R¹ = -CH₃, R²-R⁴ = -H
X = -CCl₃, R¹ = -CH₂-C₆H₅, R²-R⁴ = -H
X = -CCl₃, R¹ = -CH₂-C₆H₅, R² = -COOCH₃, R³-R⁴ = -H
X = -CCl₃, R¹ = -CH₃, R² = -COOCH₃, R³-R⁴ = -H 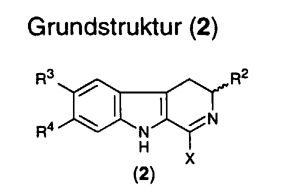
bevorzugt
X = -CF₃, R²-R⁴ = -H
X = -CCl₃, R²-R⁴ = -H
X = -CBr₃, R²-R⁴ = -H
ganz bevorzugt
X = -CF₃, R² = -COOCH₃, R³-R⁴ = -H
X = -CCl₃, R² = -COOCH₃, R³-R⁴ = -H
X = -CBr₃, R² = -COOCH₃, R³-R⁴ = -H 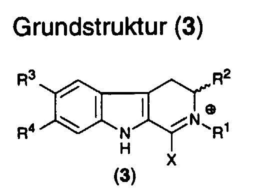 bevorzugt
   X = -CCl₃, R¹ = -C₂H₅, R²-R⁴ = -H
   X = -CCl₃, R¹ = -CH₃, R²-R⁴ = -H
   X = -CBr₃, R¹ = -C₂H₅, R²-R⁴ = -H
   X = -CBr₃, R¹ = -CH₃, R²-R⁴ = -H
   ganz bevorzugt
   X = -CCl₃, R¹ = -C₂H₅, R² = -COOCH₃, R³-R⁴ = -H
   X = -CCl₃, R¹ = -CH₃, R² = -COOCH₃, R³-R⁴ = -H
   X = -CBr₃, R¹ = -C₂H₅, R² = -COOCH₃, R³-R⁴ = -H
   X = -CBr₃, R¹ = -CH₃, R² = -COOCH₃, R³-R⁴ = -H 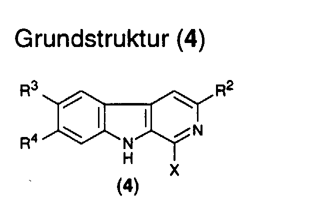
   bevorzugt
   X = -CCl₃, R²-R⁴ = -H
   X = -CBr₃, R²-R⁴ = -H
   ganz bevorzugt
   X = -CCl₃, R² = -COOCH₃, R³-R⁴ = -H
   X = -CBr₃, R² = -COOCH₃, R³-R⁴ = -H 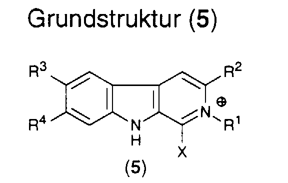
   bevorzugt
   X = -CCl₃, R¹ = -CH₃, R²-R⁴ = -H
   X = -CCl₃, R¹ = -C₂H₅, R²-R⁴ = -H
   X = -CCl₃, R¹ = -CH₂-C₆H₅, R²-R⁴ = -H
   X = -CBr₃, R¹ = -CH₃, R²-R⁴ = -H
   X = -CBr₃, R¹ = -C₂H₅, R²-R⁴ = -H
   X = -CBr₃, R¹ = -CH₂-C₆H₅, R²-R⁴ = -H
   ganz bevorzugt
   X = -CCl₃, R¹ = -CH₃, R² = -COOCH₃, R³-R⁴ = -H
   X = -CCl₃, R¹ = -C₂H₅, R² = -COOCH₃, R³-R⁴ = -H
   X = -CCl₃, R¹ = -CH₂-C₆H₅, R² = -COOCH₃, R³-R⁴ = -H
   X = -CBr₃, R¹ = -CH₃, R² = -COOCH₃, R³-R⁴ = -H
   X = -CBr₃, R¹ = -C₂H₅, R² = -COOCH₃, R³-R⁴ = -H
   X = -CBr₃, R¹ = -CH₂-C₆H₅, R² = -COOCH₃, R³-R⁴ = -H

Weitere Derivate mit beliebigen Substituenten in 4-, 5- und 8-Position des β-Carbolingerüstes sind eingeschlossen, wobei die Nummerierung des β-Carbolingerüstes wie folgt ist:

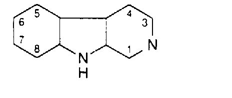

Diese Substituenten können vorzugsweise ausgewählt sein aus OH-, OCH₃-, CH₃- F-, Cl-, Br-, I-.

Ebenfalls eingeschlossen sind Derivate bei denen der Stickstoff im Indolring alkyliert (z.B. -CH₃, -C₂H₅, -C₃H₇, etc.) oder acyliert (z.B. -COCH₃, -COC₂H₅, etc.) ist.

Einige wenige der durch die Formel (I) umfaßten Verbindungen sind schon in der Literatur beschrieben worden. Jedoch wurde nicht deren hemmende Wirkung auf den Komplex I und/oder II der Atmungskette erkannt.

Diese bekannten Verbindungen sind:

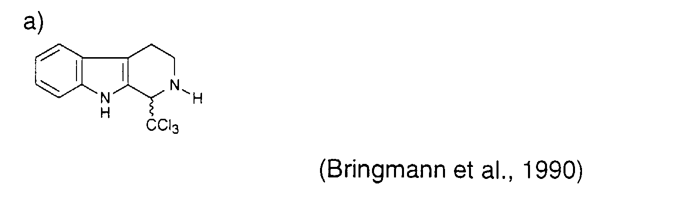

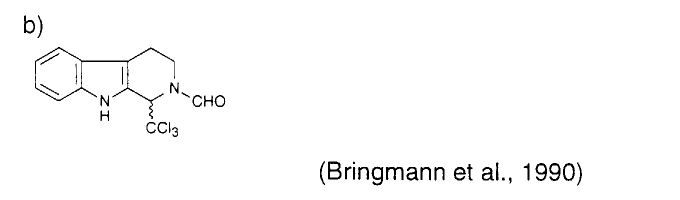

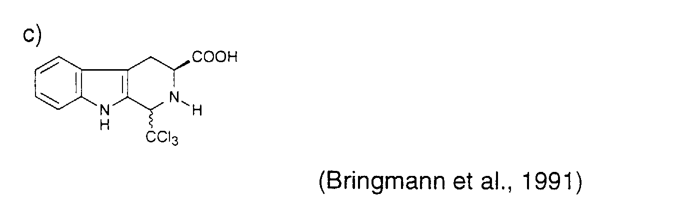

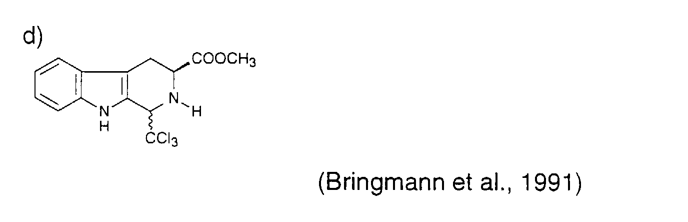

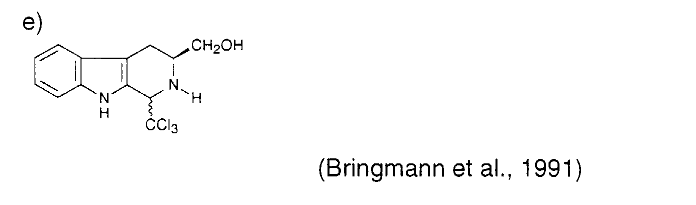

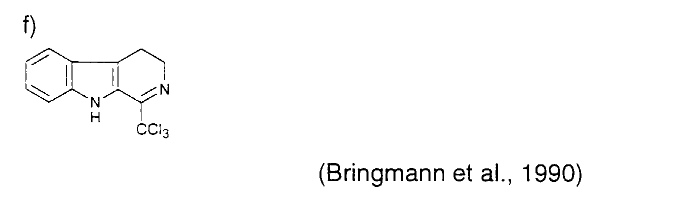

Weitere Verbindungen, die teilweise die obigen Definitionen erfüllen, aber bei denen die hemmende Wirkung auf die Atmungskette bisher nicht erkannt wurde, sind beschrieben in:
- Julia et al., Bulletin de las Societe Chimique de France, Nr. 4, 1973, S. 1424-1426
- Bringmann et al., Liebigs Annalen der Chemie, 1991, S. 1189-1194
- GB-A-975 835
- Vecchietti et al., J. of Medicinal Chemistry, Bd. 34, 1991, S. 2624-2633
- Chemical Abstracts, Bd. 114, Nr. 5, 1991, 42 368d
- Maki et al., J. of Fluorine Chemistry, 43 (1989), S. 189-206
- Chemical Abstracts, Bd. 106, Nr. 3, 1987, 18 907t
- Chemical Abstracts, Bd. 110, Nr. 1, 1989, 2 895j.

Besonders bevorzugt sind Verbindungen mit der folgenden Formel, da sie sich durch eine besonders gute Hemmwirkung auszeichnen: 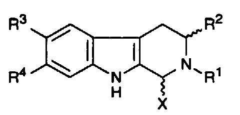
R¹ = Alkyl mit C₁-C₁₈, z.B. Methyl, Ethyl, Propyl, *n*-Butyl, *iso*-Butyl, *tert*.-Butyl etc.
R² - R⁴ und X haben die oben bei der Struktur (**I**) definierten Bedeutungen

Davon sind besonders bevorzugt:
X = -CCl₃, R¹ = -CH₃, R²-R⁴ = -H
X = -CCl₃, R¹ = -C₂H₅, R²-R⁴ = -H
X = -CCl₃, R¹ = -CH₃, R² = COOCH₃, R³-R⁴ = -H
X = -CCl₃, R¹ = -C₂H₅, R² = COOCH₃, R³-R⁴ = -H
X = -CBr₃, R¹ = -CH₃, R²-R⁴ = -H
X = -CBr₃, R¹ = -C₂H₅, R²-R⁴ = -H
X = -CBr₃, R¹ = -CH₃, R² = COOCH₃, R³-R⁴ = -H
X = -CBr₃, R¹ = -C₂H₅, R² = COOCH₃, R³-R⁴ = -H

Auch Verbindungen mit der folgenden Formel sind bevorzugt:

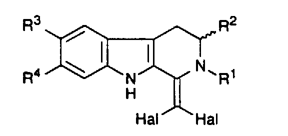

Hal hat dabei die Bedeutung von F, Cl, Br oder I, die gleich oder verschieden sein können.

Die Substituenten R¹, R², R³ und R⁴ haben die bei der Struktur (**I**) definierten Bedeutungen.

Davon sind besonders bevorzugt:
HaI = -Cl, R¹-R⁴ = -H
HaI = -Cl, R¹ = -OH₃, R²-R⁴ = -H
HaI = -Cl, R¹ = -H, R² = -COOCH₃, R³-R⁴ = -H
HaI = -Cl, R¹ = -CH₃, R² = -COOCH₃, R³-R⁴ = -H
HaI = -Br, R¹-R⁴ = -H
HaI = -Br, R¹ = -CH₃, R²-R⁴ = -H
HaI = -Br, R¹ = -CHO, R²-R⁴ = -H
HaI = -Br, R¹ = -H, R² = -COOCH₃, R³-R⁴ = -H
HaI = -Br, R¹ = -CH₃, R² = -COOCH₃, R³-R⁴ = -H

Aus der Vielzahl der durch die Grundstrukturen abgedeckten Verbindungen seien nun weitere genannt, die sich durch eine besonders gute Wirksamkeit ausgezeichnet haben, wobei hervorgehoben werden muß, daß alle unter die Grundstrukturen fallenden Verbindungen die oben genannte Aufgabe lösen:
Grundstruktur (**1**), X = -CCl₃, R¹ = -CH₂-CH=CH₂, R²-R⁴ = -H
Grundstruktur (**1**), X = -CCl₃, R¹-R⁴ = -H, Methylgruppe in 8-Position
Grundstruktur (**1**), X = -HCCl₂, R¹-R⁴ = -H
Grundstruktur (**1**), X = -HCCl₂, R¹ = -COCCl₃, R²-R⁴ = -H
Grundstruktur (**1**), X = -HCCl₂, R¹ = -COCH₂Cl, R²-R⁴ = -H

Synthetisch lassen sich die 1-Trichlormethylheterocyclen durch Pictet-Spengler-Ringschluß aus 3-Indolylethylaminen und Chloral oder durch Bischler-Napieralski-Cyclisierung von 3-lndolylethylamin-trichloracetamiden erhalten.

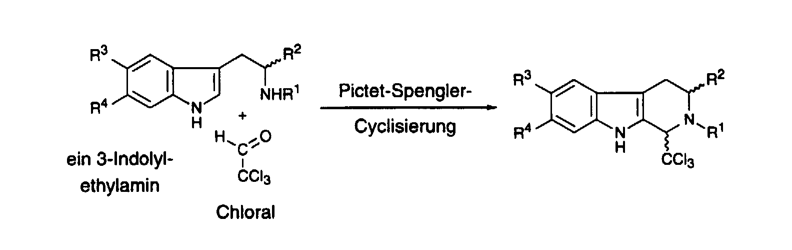

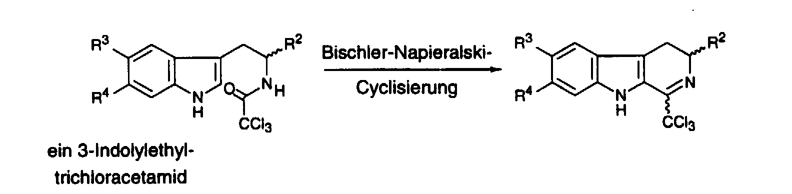

Die so erhaltenen Grundstrukturen können dann durch Reduktion und Oxidation mit geeigneten Reagenzien in die jeweils anderen Grundstrukturen überführt werden. Die Funktionalitäten R¹, R², R³ und R⁴ können durch Verwendung entsprechender Edukte (d.h. entsprechender Amin- bzw. Amidderivate) eingeführt werden. Sie lassen sich ggf. auch nachträglich einbauen oder noch weiter modifizieren. Ganz analog lassen sich auch die Derivate mit Funktionalitäten in 4-, 5- und 8-Position erhalten. β-Carbolinderivate mit anderen Halogenatomen als Chlor lassen sich prinzipiell nach den gleichen Verfahren darstellen.

Es ist damit zu rechnen, daß die Verbindungen mit Grundstruktur (1) durch enzymatische und/oder nichtenzymatische Oxidationsreaktionen in die Grundstrukturen (**2**),(**3**) und/oder (**4**),(**5**) biotransformiert werden. Biotransformationsreaktionen sind auch bezüglich der funktionellen Gruppen in Betracht zu ziehen.

Diese neuartigen Chloral-abgeleiteten Heterocyclen zeichnen sich durch das Vorhandensein einer großen, lipophilen Halogen-enthaltenden Gruppe aus. Wahrscheinlich aufgrund dieser lipophilen Eigenschaft ist das Überwinden der Blut-Hirn-Schranke erleichtert.

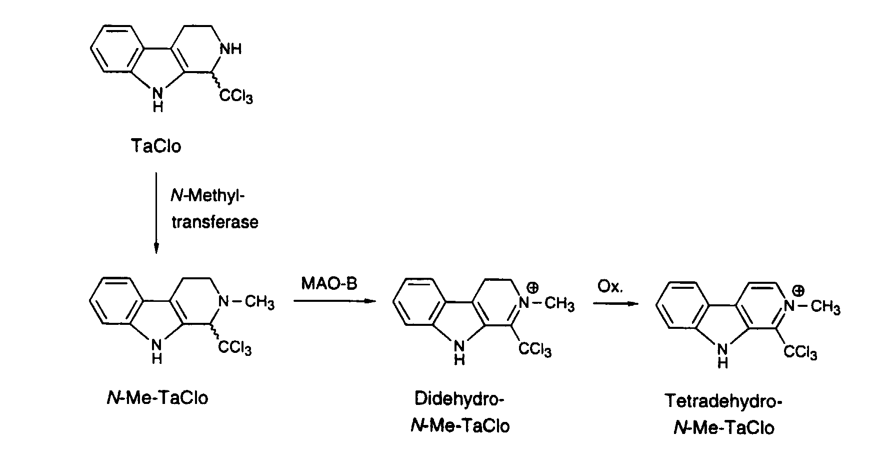

### Abbildung 1. Möglicher Wirkmechanismus am Beispiel von TaClo.

Die Erfinder haben den unerwarteten Befund erhalten, daß das von Tryptamin ("Ta") und Chloral ("Clo") abgeleitete 1-Trichlormethyl-1,2,3,4-tetrahydro-β-carbolin "TaClo" (vgl. Abbildung 1) eine sehr starke Hemmwirkung gegenüber Komplex I der mitochondrialen Atmungskette besitzt. Eine 85proz. Hemmung konnte mit TaClo bei 500 µM beobachtet werden, während viele bekannte Hemmstoffe erst bei 10-20 fach höheren Konzentrationen eine vergleichbare Hemmung aufweisen. Weiterhin konnte gezeigt werden, daß potentielle TaClo-Metabolite, so z.B. die *N*-methylierte Form (*N*-Me-TaClo) bzw. die oxidierten Formen (Didehydro-*N*-Me-TaClo und Tetradehydro-*N*-Me-TaClo) aber auch Dichlormethylen-TaClo, ein potentieller Metabolit, noch wesentlich stärker wirkende Spezies sind. Vergleichbare Ergebnisse wurden auch mit den von der Aminosäure Tryptophan abgeleiteten Kondensationsprodukten (Grundstruktur (**1**), R² = COOH bzw. R² = COOCH₃, R¹ = R³ = R⁴ = H) erzielt. Die *N*-methylierten Verbindungen zeigen bereits bei Konzentrationen < 250 µM in Gehimhomogenaten eine 100proz. Hemmung des Komplexes I. Eine überdurchschnittliche Hemmwirkung wurde auch für β-Carbolinderivate beobachtet, die anstelle der 1-Tdchlormethylgruppe z.B. eine 1-Tribrommethylgruppe, 1-Trifluormethylgruppe oder eine 1-Dichlormethylenfunktion besitzen. Im Fall z.B. von TaClo ist diese Wirksamkeit sehr spezifisch: Die beobachtete Hemmung des Komplexes I beschränkt sich lediglich auf die vom Komplex I katalysierte Reaktion der CoQ-Reduktion, beeinflußt jedoch nicht die NADH-Oxidation (s. Tabelle 2).

Die oben als bevorzugt erwähnten N-Alkylverbindungen sind über *N*-Alkylierung herstellbar. So wird z.B. für die Herstellung der alkylierten TaClo-Derivate TaClo-Hydrochlorid in Alkohol, bevorzugt Methanol, gelöst und mit NaHCO₃ und Alkylhalogenid, Alkylsulfat oder Alkylsulfonat umgesetzt. Nach Einengen der Reaktionslösung erfolgt eine Chromatographie, bevorzugt an Kieselgel. Nach Zugabe von einer alkoholischen Mineralsäure-Lösung fällt das N-alkylierte Produkt aus. Dieses Verfahren eignet sich natürlich auch zur Darstellung der Verbindungen mit der Grundstruktur (**3**) oder (**5**), sofern Ausgangsverbindungen mit der Grundstruktur (**2**) oder (**4**) eingesetzt werden.

Die *N*-Alkylverbindungen lassen sich auch durch Reduktion mit Reduktionsmitteln, wie z.B. LiAlH₄ herstellen. dies ist überraschend, da zu erwarten wäre, daß die X-Gruppe, die bevorzugt CCl₃ oder CBr₃ ist, ebenfalls reduziert würde. So läßt sich z.B. ein alkyliertes TaClo-Derivat aus der entsprechenden Acyl-Verbindung und einem Reduktionsmittel herstellen.

Sehr gut wirken auch TaBro-Derivate, die durch Reaktion von einem Tryptamin-Derivat mit Bromal gewonnen werden können. Eine solche Verbindung hat die Formel 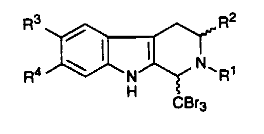 bevorzugt mit:
R^{l}-R⁴ = -H
weiter bevorzugt mit:
R¹ = -CHO, R²-R⁴ = -H
R¹ = -CH₃, R²-R⁴ = -H
R¹ = -C₂H₅, R²-R⁴ = -H
R¹ = -CH₂-C₆H₅, R²-R⁴ = -H
R¹ = -H, R² = -COOCH₃, R³-R⁴ = -H
R¹ = -CH₃, R² = -COOCH₃, R³-R⁴ = -H
R¹ = -C₂H₅, R² = -COOCH₃, R³-R⁴ = -H
R¹ = -CH₂-C₆H₅, R² = -COOCH₃, R³-R⁴ = -H

Die meisten β-Carbolin-Verbindungen fallen bei der Synthese als Racemate an. Diese Enantiomeren-Gemische haben oft pro Gramm Verbindung eine geringere Wirksamkeit als die stereochemisch einheitlichen Wirkstoffe. So geht die Bemühung dahin, die Enantiomere zu trennen. Dies kann durch bekannte Verfahren geschehen oder durch die speziellen Verfahren, die in den nachfolgenden Beispielen angegeben sind.

Eine Verbindung mit der Grundstruktur (**1**), bevorzugt TaClo (R¹, R², R³, R⁴ = H), läßt sich bevorzugt in großen Mengen über den Umweg der *N*-Formyl-Verbindung besonders elegant herstellen. Dabei wird im Falle der Herstellung von TaClo Tryptamin in Ameisensäure mit Chloral umgesetzt. Nach Erwärmen wird ein Alkohol, bevorzugt Methanol, zugegeben. Nach Kristallisation des Produkts (*N*-Formyl-TaClo) wird dieses in verdünnter Mineralsäure, bevorzugt Salzsäure, und Alkohol, bevorzugt Methanol, suspendiert und erhitzt. Nach Einengen der Flüssigkeitsmenge wird der erhaltene Feststoff abfiltriert.

Zur Herstellung der vollaromatischen β-Carbolin-Derivate mit der Grundstruktur (**4**) bzw. (**5**) wird bevorzugt von den Grundstrukturen (**1**),(**2**) bzw. (**3**) ausgegangen und mit Oxidationsmitteln, bevorzugt KMnO₄, oder Dehydrierungskatalysatoren oxidiert.

Die auch bevorzugt verwendeten N-Acylverbindungen werden durch Umsetzung der entsprechenden Verbindungen der Grundstrukturen (**1**),(**2**),(**3**),(**4**) oder (**5**) mit Acylierungsreagenzien, bevorzugt Säurehalogeniden oder -anhydriden umgesetzt, z.B.

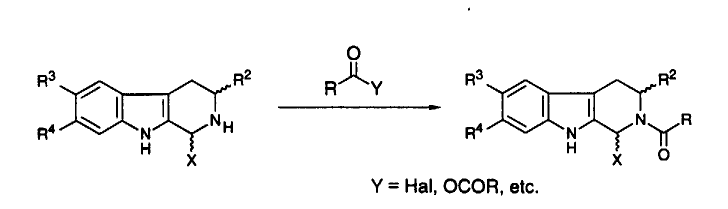

Bei der Synthese von erfindungsgemäßen Verbindungen, bei denen der Rest X eine -CHHal₂-Gruppe sein soll, geht man bevorzugt von der entsprechenden Verbindung mit einer CHal₃-Gruppe aus und reduziert diese mit Reduktionsmitteln, z.B. Natriumborhydrid in absolutem Methanol oder mit Wasserstoff und Pd/C als Katalysator in 80proz. Methanol. Altemativ geht auch eine *de-novo*-Synthese aus z.B. Dihalogenacetaldehyd und den entsprechenden Tryptamin-Derivaten.

Beispielhaft sei auf die nachfolgenden Beispiele und die darin enthaltenen Formeln der Verbindungen verwiesen.

Die vorliegende Erfindung wird nun mit Bezug auf die folgenden Beispiele näher beschrieben.

### Beispiele

### Beispiel 1

Synthetische Zugänge zur Grundstruktur (**1**):
1.1. Die Pictet-Spengler-Kondensation könnte auch bei einer *in-vivo*-Entstehung von 1-Trichlormethyl-β-carbolinderivaten im Säugerorganismus der zentrale Schritt sein: 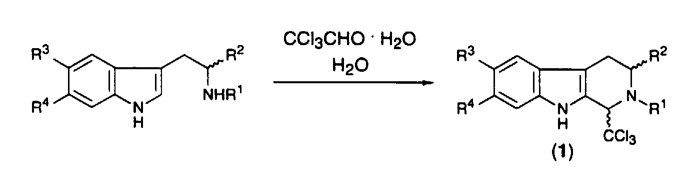 In 5 ml Wasser werden 100 mg (0.62 mmol) Tryptamin und 205 mg (1.24 mmol) Chloralhydrat gelöst und 32 h bei 40°C gerührt. Das Lösungsmittel wird zur Trockene eingedampft, der Rückstand an Kieselgel (Laufmittel: Petrolether/Chloroform = 1 : 3 v/v) chromatographiert. Die erhaltene Fraktion wird mit methanolischer HCI versetzt, das Hydrochlorid aus Methanol/Ether umkristallisiert, und man erhält 82 mg (0.25 mmol, 40.5%) TaClo (Hydrochlorid) in Form farbloser, pulvriger Kristalle, die sich oberhalb von 230°C zersetzen.
1.2. Für präparative Zwecke in größerem Maßstab eignet sich besser die Umsetzung der Amine mit Chloral in wasserfreien Medien wie z.B. Toluol, Dioxan oder Trifluoressigsäure: 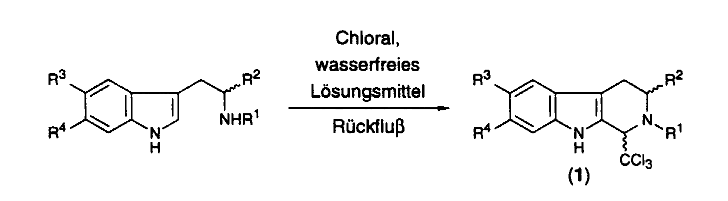 Eine Lösung von 100 mg (0.62 mmol) Tryptamin und 0.5 ml (5.1 mmol) Chloral in 5 ml Dioxan wird 5 min unter Rückfluß erhitzt. Nach Erkalten wird das Lösungsmittel abdestilliert, der Rückstand an Kieselgel (Laufmittel: Dichlormethan) chromatographiert, das Eluat mit methanolischer HCI versetzt und vom Lösungsmittel befreit. Nach Umkristallisieren des Rohproduktes aus MethanoVEther erhält man 106 mg (0.33 mmol, 52.1%) TaClo (Hydrochlorid) in Form farbloser, pulvriger Kristalle, die sich oberhalb von 230°C zersetzen.
1.3. Eine deutliche Verbesserung ergibt sich durch längeres Erhitzen einer Lösung von Amin und Chloral in Ameisensäure auf 70°C: 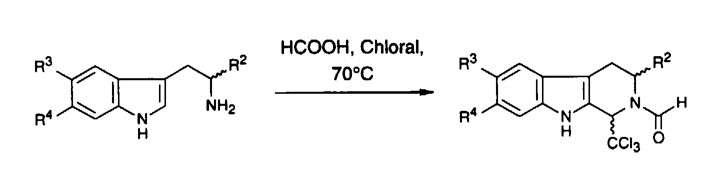
   Diese Reaktion ist besonders gut geeignet für die Darstellung großer Mengen von sehr reinem TaClo: 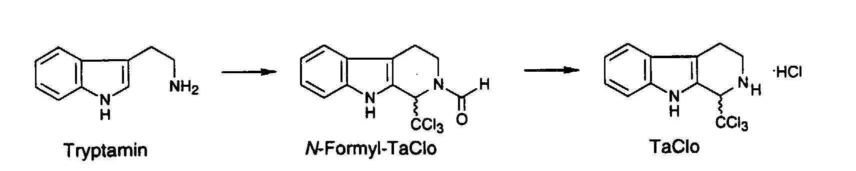
   a) Zu einer Lösung von 100 g (624 mmol) Tryptamin (R² = R³ = R⁴ = H) in 150 ml Ameisensäure werden unter Rühren 91.4 ml (137 g; 936 mmol) Chloral zugetropft. Anschließend wird die Reaktionslösung zwei Stunden auf 70°C erwärmt. Nach dem Abkühlen werden 250 ml Methanol zugegeben. Man läßt zwei Tage bei Raumtemperatur stehen, filtriert dann das ausgefallene Rohprodukt ab und kristallisiert dieses aus Methanol. Man erhält 145 g (73 %) *N*-Formyl-TaClo (R² = R³ = R⁴ = H) als beige Kristalle mit Schmp. 223°C (Zers.).
   b) 18 g *N*-Formyl TaClo werden in 300 ml Methanol und 60 ml 35proz. Salzsäure suspendiert und 2.5 Stunden zum Rückfluß erhitzt. Anschließend wird die Reaktionslösung im Vakuum auf ein Viertel eingeengt und der erhaltene Feststoff abfiltriert. Man erhält 18 g (97 %) TaClo (Hydrochlorid) als farbloses Kristallpulver mit Schmp. 230°C (Zers.).
1.4 Tetrahydro-β-carbolinderivate lassen sich auch durch Reduktion z.B. mit Natriumcyanoborhydrid aus Derivaten mit der Grundstruktur (2) bzw. (3) erhalten: 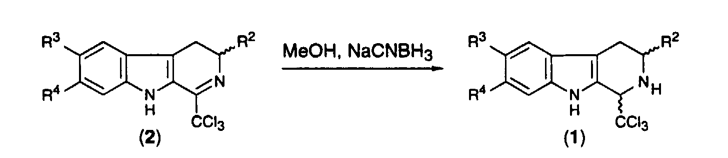 Zu einer Lösung aus 45 mg (0.16 mmol) 1,2-Didehydro-TaClo in 2 ml einer Mischung aus gleichen Teilen wasserfreiem Chloroform und wasserfreiem Methanol gibt man bei pH 4 und 0.5°C 11 mg (0.18 mmol) Natriumcyanoborhydrid. Nach 2 h quencht man mit etwas Aceton, verdampft das Lösungsmittel und chromatographiert an Kieselgel (Laufmittel: Chloroform). Man versetzt mit methanolischer HCI und erhält nach Kristallisieren aus Methanol/Ether 10.3 mg (0.03 mmol, 20.2%) TaClo (Hydrochlorid), welches sich oberhalb von 230°C zersetzt.

### Beispiel 2

Synthetische Zugänge zur Grundstruktur (**2**) bzw. (**3**):
2.1. Durch Umsetzung des jeweiligen Tryptamin-trichloracetamids mit Phosphorpentoxid in siedendem Xylol (Bischler-Napieralski-Reaktion) erhält man Derivate mit der Grundstruktur (**2**): 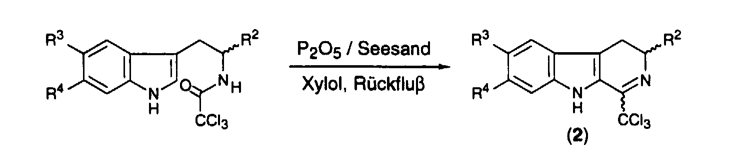 Zu einer Lösung von 2 g (6.55 mmol) Tryptamin-trichloracetamid in 150 ml wasserfreiem Xylol gibt man bei 110°C in mehreren Portionen ein Gemisch aus 17 g (120 mol) Phosphorpentoxid und 34 g Seesand. Man erhitzt bei starkem Rühren mit einem KPG-Rührwerk 24 h unter Rückfluß. Nach dem Abkühlen filtriert man ab, wäscht mit Petrolether nach und hydrolysiert den Rückstand vorsichtig mit 200 g Eis. Unter Kühlung versetzt man die so erhaltene stark saure Lösung mit festem Natriumhydroxid bis pH 5 und extrahiert mit Essigester. Die vereinigten organischen Phasen werden über Magnesiumsulfat getrocknet, das Lösungsmittel eingedampft und der Rückstand an Kieselgel (Laufmittel: Dichlormethan) chromatographiert. Man erhält nach Kristallisation aus Methanol/Dichlormethan 50 mg (0.17 mmol, 2.6%) 1,2-Didehydro-TaClo in Form hellbeiger Nadeln, die sich oberhalb von 156°C zersetzen.
2.2. Derivate mit der Grundstruktur (**2**) bzw. (**3**) erhält man auch durch Oxidation der 1,2,3,4-Tetrahydro-β-carbolinderivate (Grundstruktur (**1**)) mit diversen Oxidationsmitteln, z.B. mit Kaliumpermanganat in Tetrahydrofuran oder in Aceton: 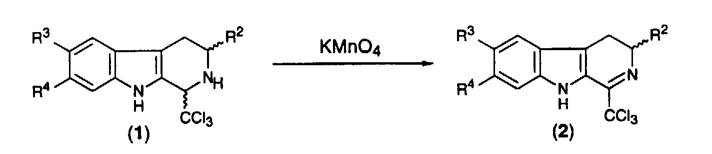 Ähnlich lassen sich nun auch 1-Trichlommethyl-*N*-alkyldihydro-β-carboliniumsalze **3** gewinnen: 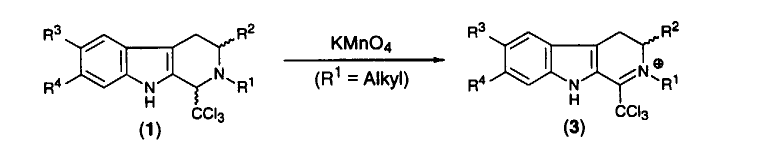 Eine Lösung von 70 mg (0.22 mmol) TaClo (Hydrochlorid) in 15 ml wasserfreiem Tetrahydrofuran wird mit 120 mg (0.76 mmol) Kaliumpermanganat versetzt und 3.5 h bei Raumtemperatur gerührt. Vom Niederschlag wird abfiltriert, das Filtrat zur Trockene eingedampft und an Kieselgel chromatographiert (Laufmittel: Chloroform/Petrolether = 3:2 v/v) Nach Umkristallisation aus Dichlormethan/Ether erhält man 8.2 mg (0.029 mmol, 13.3%) 1,2-Didehydro-TaClo in Form hellbeiger Nadeln, die sich oberhalb von 156°C zersetzen.
2.3. Durch Umsetzung von N-Alkylverbindungen der Grundstruktur (**1**) mit Wasserstoffperoxid erhält man das N-Oxid, welches dann mit Trifluoressigsäureanhydrid in das 1,2-Didehydroderivat überführt wird:

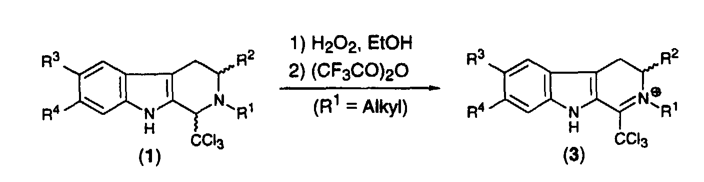

### Beispiel 3

Synthetische Zugänge zur Grundstruktur (**4**) bzw. (**5**):
3.1. Die vollaromatischen 1-Trichlormethyl-β-carboline können durch Oxidation der Derivate mit der Grundstruktur (**1**), (**2**) bzw. (**3**) oder (4) bzw. (**5**) mit verschiedenen Oxidationsmitteln (z.B. KMnO₄) oder Dehydrierungskatalysatoren (z.B. Pt) erhalten werden:

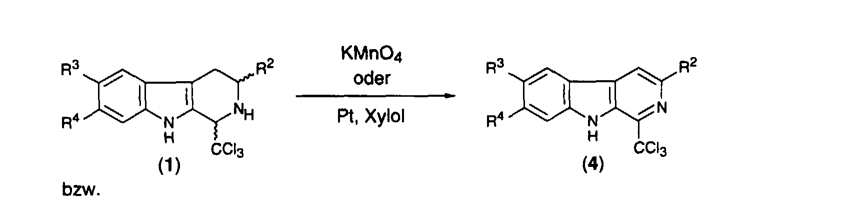

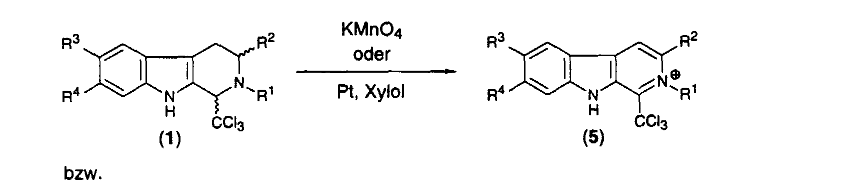

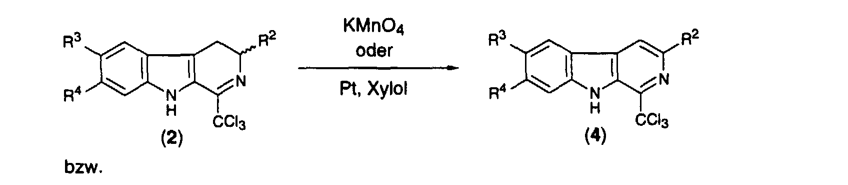

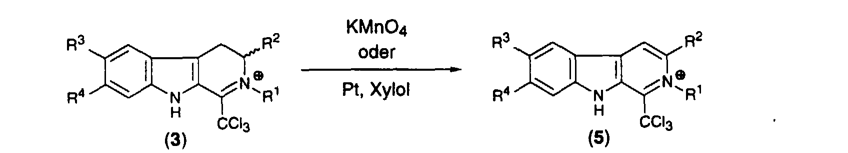

Eine Suspension von 200 mg (0.61 mmol) TaClo (Hydrochlorid) in 25 ml Aceton und 6 ml Chloroform wird auf 50°C erhitzt und mit 320 mg (2.02 mmol) Kaliumpermanganat versetzt. Nach 30 min wird die Reaktionslösung filtriert, vom Lösungsmittel befreit und an Kieselgel (Laufmittel: Methyl-*tert*.-butylether/Petrolether = 1: 2 v/v) chromatographiert. Das Eluat wird zur Trockene eingeengt. Man erhält 11 mg (0.04 mmol, 6%) 1-Trichlormethyl-β-carbolin als beigen Feststoff, der sich oberhalb von 110°C zersetzt.

### Beispiel 4

### Variation der funktionellen Gruppen

Variation der Restes R¹ :
4.1. Die *N*-Alkylierung gelingt u.a. mit Alkylhalogeniden, -sulfaten oder Alkylsulfonaten (z.B. Trifluormethylsulfonsäurealkylestern) z.B. in Gegenwart von Natriumhydrogencarbonat in Methanol oder in Dimethylsulfoxid: 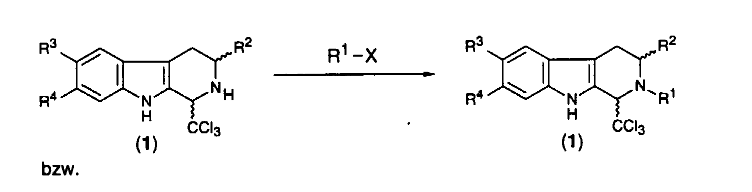 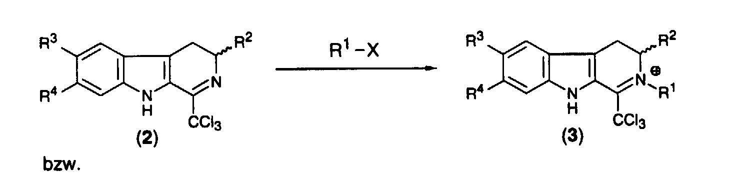 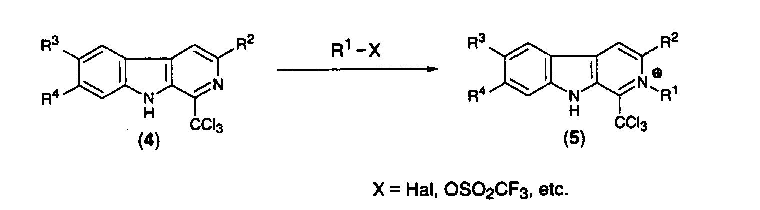
   Zu einer Lösung von 1.0 g (3.07 mmol) TaClo (Hydrochlorid) in 40 ml Methanol werden 1.0 g (11.9 mmol) NaHCO₃ und 2 ml (4.34 g, 30.7 mmol) Methyliodid gegeben. Danach wird 60 Stunden bei Raumtemp. unter Lichtausschluß gerührt. Anschließend wird die Reaktionslösung zur Trockene eingeengt und der Rückstand mit einer Mischung aus Methyl-*tert*.-butylether und Petrolether (1 : 2 v/v) als Fließmittel an Kieselgel chromatographiert. Das Eluat wird mit einem geringen Überschuß methanolischer HCI-Lösung versetzt. Das dabei ausfallende N-methylierte TaClo (Hydrochlorid) wird vom Lösungsmittel befreit und im Ölpumpenvakuum getrocknet. Man erhält 0.67 g (1.97 mmol, 64%) der N-methylierten Verbindung in Form eines leicht gelblichen amorphen Pulvers welches sich beim Erhitzen auf 96°C zersetzt.
   Trotz Reduktionempfindlichkeit der CCl₃-Gruppe lassen sich *N*-Alkylverbindungen überraschenderweise auch aus den *N*-Acylderivaten mit Reduktionsmitteln wie z.B. Lithiumaluminiumhydrid erhalten: 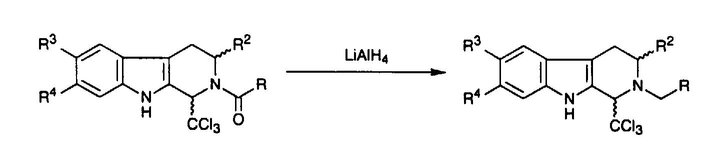 In einer Lösung von 50 mg (0.16 mmol) von *N*-Formyl-TaClo in 5 ml trockenem Tetrahydrofuran werden 25 mg AlCl₃ suspendiert. Nach 15 min Rühren bei Raumtemp. versetzt man mit einem geringen Überschuß Lithiumaluminiumhydrid und rührt weitere 15 min. Dann wird mit Wasser hydrolysiert und mit 10 ml Ether versetzt. Nach Filtration wird die Lösung bis zur Trockene eingeengt. Der Rückstand wird mit einer Mischung aus Methyl-*tert*.-butylether und Petrolether (1 : 2 v/v) als Fließmittel an Kieselgel chromatographiert. Das Eluat wird mit einem geringen Überschuß an methanolischer HCI-Lösung versetzt und bis zur Trockene eingeengt. Man erhält 31 mg (0.09 mmol, 60%) der *N*-Methyl-Verbindung als Hydrochlorid in Form eines leicht gelblichen amorphen Pulvers welches sich beim Erhitzen auf 96°C zersetzt.
4.2. Die *N*-Acylverbindungen erhält man durch Umsetzung der Derivate mit der Grundstruktur (**1**) mit Acylierungsreagenzien wie z.B. Säurehalogeniden oder -anhydriden: 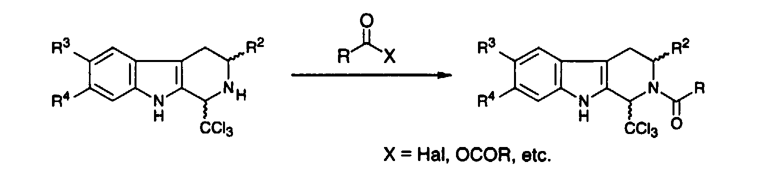
   Zu einer Lösung von 100 mg (0.31 mmol) TaClo (Hydrochlorid) und 170 µl (124 mg, 1.23 mmol) Triethylamin in 10 ml Dichlormethan gibt man bei 0°C eine Lösung von 66 µl (72 mg, 0.92 mmol) Acetylchlorid in 2 ml Dichlormethan. Nach 20 min. Rühren bei Raumtemp. wäscht man zweimal mit je 5 ml 1 M Salzsäure und dann bis zur neutralen Reaktion mit Wasser. Man trocknet die organische Phase über Magnesiumsulfat und engt im Vakuum ein. Nach Umkristallisation aus Methanol erhält man 92 mg (0.28 mmol, 90%) *N*-Acetyl-TaClo als farblose Kristalle, die sich oberhalb von 215°C zersetzen.

Variation des Restes R²:
4.3. Die gewünschten Derivate mit Esterfunktion in 3-Stellung erhält man am einfachsten, wenn die entsprechenden Tryptophanester mit Chloral kondensiert werden. Aber auch eine nachträgliche Umwandlung z.B. durch Veresterung oder sonstige Umwandlung (z.B. Amidierung, etc.) ist möglich: 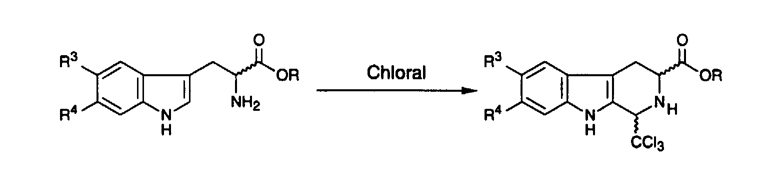
   Eine Lösung aus 530 mg (2.09 mmol) L-Tryptophan-methylester (Hydrochlorid) und 2.44 ml (25.0 mmol) Chloral in 5.3 ml Ameisensäure wird 15 h bei Raumtemp. gerührt. Nach Säulenchromatographie des Eindampfrückstandes an Kieselgel (Laufmittel: Chloroform/Methanol) und Umkristallisieren aus Aceton/Petrolether erhält man 385.5 mg (0.81 mmol) des chromatographisch schneller eluierenden *cis*-Diastereomeren als weiße watteartige Kristalle und 312.3 mg (1.22 mmol) des langsameren *trans*-Diastereomeren in Form nadelartiger Kristalle. Gesamtausbeute 706.8 mg (2.03 mmol, 83.7%).
4.4 Entsprechende Alkohole R² = -CH₂-OH entstehen bei Behandlung der Ester R² = -COOR mit Reduktionsmitteln wie z.B. Natriumborhydrid; wahlweise kann die Reaktion auch so geführt werden, daß zusätzlich die CCl₃-Gruppe reduziert wird (vgl. 5.2). 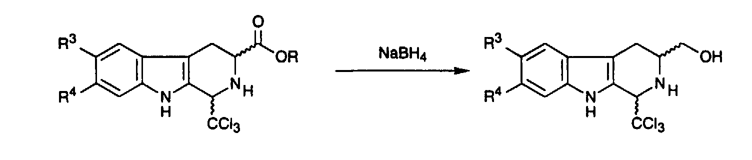
   Eine Lösung von 150 mg (0.43 mmol) des sich von L-Tryptophanmethylester ableitenden (1*R*, 3*S*)-Heterocyclus in 40 ml Methanol wird unter Kühlung und Rühren portionsweise mit 2.3 g (60.1 mmol) Natriumborhydrid versetzt. Die Lösung wird noch 15 min bei Raumtemp. gerührt und dann mit verdünnter Salzsäure auf pH 6-7 gebracht. das Lösungsmittel wird abgedampft, der Rückstand mit Isopropanol digeriert und das Filtrat an Kieselgel (Laufmittel: Chloroform/Methanol = 15 : 1 v/v) chromatographiert. Man erhält dabei 52 mg (0.16 mmol, 37%) des (1*R*, 3*S*)-konfigurierten Alkohols, der sich oberhalb von 123°C zersetzt. [a]_{D}²⁵ = -71.4 (c = 0.5 in Methanol).
   Auch die primäre Alkoholfunktion kann ihrerseits nach Standardverfahren weiter modifiziert werden u.a. durch Oxidation zum Aldehyd, *O*-Alkylierung, *O*-Acylierung, Dehydratisierung, etc.).
4.5 Amide R² = COONR'R" erhält man bei Behandlung der Ester R² = COOR mit einer Lösung des Amins HNR'R" in Methanol (z.B. R = CH₃, R' = R" = H), sowie durch Kondensation entsprechender Tryptophanamide mit Chloral: 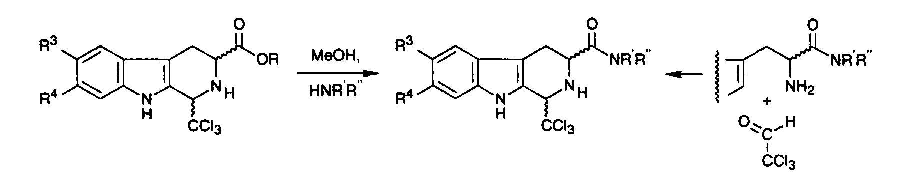
   Auch die Amidfunktion kann durch Standardverfahren weiter verändert werden, u.a. durch Reduktion, Dehydratisierung, *O*- oder *N*-Alkylierung, etc.

Variation der Reste R³ und/oder R⁴:
4.6. In 6- und/oder 7-Stellung substituierte (z.B. alkoxylierte) Derivate lassen sich sehr einfach aus dem entsprechend substituierten Tryptaminderivat und Chloral erhalten (z.B. R³ = OCH₃, R⁴ = H oder R³ = H, R⁴ = OCH₃): 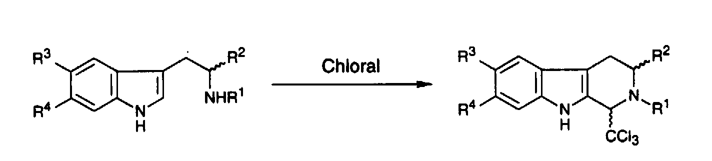 z.B. R³, R⁴ = OCH₃, OC₂H₅, OH, NMe₂ etc.
   Zu einer Suspension aus 200 mg (0.88 mmol) 5-Methoxy-tryptamin (Hydrochlorid) in 7 ml Toluol gibt man 1.5 ml (2.27 g, 15.4 mmol) Chloral und erhitzt 150 min unter Rückfluß. Anschließend wird die Reaktionslösung bis zur Trockene eingeengt. Nach Chromatographie an Kieselgel (Laufmittel: Chloroform/Methanol = 7 : 1 v/v) wird das erhaltene Öl in das Hydrochlorid überführt. Man erhält nach Umkristallisation aus MethanoVEther 238 mg (0.67 mmol, 76.4%) 1-Trichlormethyl-6-methoxy-1,2,3,4-tetrahydro-β-carbolin als leicht bräunliche, quaderförmige Kristalle, die sich oberhalb 166°C zersetzen.
   Die so erhaltenen Heterocyclen lassen sich durch Standardverfahren weiter verändem, z.B. durch *O*-Alkylierung, *O*-Acylierung, *O*-Dealkylierung, Oxidation, etc. Beispielsweise gelingt so die Darstellung freier Hydroxyverbindungen besonders günstig durch Spaltung der entsprechenden Methylether z.B. mit Lewis-Säuren wie Bortribromid oder mit Protonensäuren: 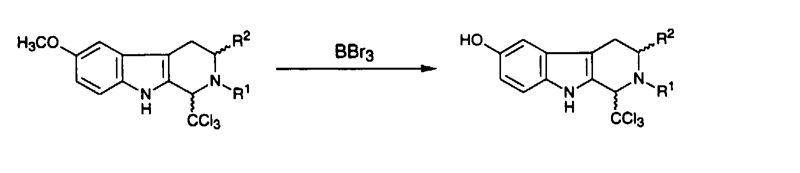 150 mg (0.42 mmol) 6-Methoxy-1,2,3,4-tetrahydro-β-carbolin werden in 10 ml Dichlormethan aufgenommen, auf -65°C abgekühlt und mit 300 µl (792 mg, 3.16 mmol) Bortribromid versetzt. Die Reaktionsmischung läßt man langsam auf Raumtemp. erwärmen und rührt dann bei Raumtemp. bis kein weiterer Umsatz mehr festgestellt werden kann (DC-Kontrolle, Kieselgelplatte, Laufmittel: Chloroform/Methyl-*tert*.-butylether = 3 : 1 v/v). Nach nochmaligem Abkühlen auf 0°C wird durch Zugabe von Wasser überschüssiges Bortribromid hydrolysiert. Die Reaktionsmischung wird nachfolgend dreimal mit Ether extrahiert, und die vereinigten Etherphasen werden mit Magnesiumsulfat getrocknet. Nach Abdestillieren des Lösungsmittels erhält man einen braunen Feststoff, der zur Reinigung an Kieselgel (Laufmittel: Methyl-*tert*.-butylether) chromatographiert wird. Man erhält nach Enffernen des Lösungsmittels 61 mg (0.20 mmol, 47.5%) 1-Trichlormethyl-6-hydroxy-1,2,3,4-tetrahydro-β-carbolin, welches sich beim Erhitzen über 124°C zersetzt.

### Beispiel 5

### Variation der Halogen-enthaltenden Gruppe X

5.1 Die Dehydrohalogenierung der 1-Trichlormethylgruppe zur 1-Dichlormethylenfunktion findet sehr leicht unter Baseneinfluß statt z.B. beim Erhitzen mit methanolischer Kaliumhydroxidlösung; altemativ läßt sich die Dichlormethylenstruktur (und ähnliche Partialstrukturen) auch durch Bischler-Napieralski-Synthese aus dem Dichloracetamid gewinnen: 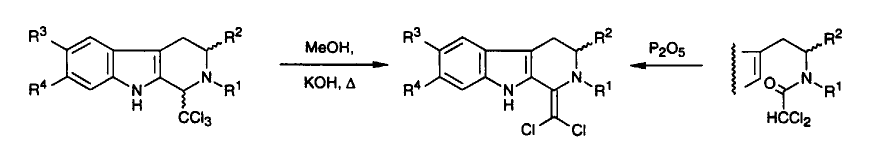
   a) 500 mg (1.53 mmol) TaClo werden in 50 ml Methanol suspendiert und mit einer Lösung von 0.3 g (5.35 mmol) Kaliumhydroxid in 15 ml Methanol versetzt. Nach 5 min. Erhitzen auf Rückflußtemp. wird die gelbe Lösung abgekühlt, mit 3 g Kieselgel versetzt und vom Lösungsmittel befreit. Zur Abtrennung von anorganischen Salzen wird das so erhaltene Pulver auf eine kurze Kieselgelsäule aufgegeben und mit Methyl-*tert*.-butylether/Petrolether = 1: 2 v/v eluiert. Das Eluat wird mit methanolischer HCI versetzt, vom Lösungsmittel befreit und aus wenig Methanol umkristallisiert. Man erhält 249 mg (0.85 mmol, 56%) Dichlormethylen-TaClo (Hydrochlorid) als orange Kristalle, die sich oberhalb von 180°C zersetzen.
   b) 1 g (3.15 mmol) N-Formyl-TaClo werden in 50 ml Methanol suspendiert und mit einer Lösung von 0.6 g (10.70 mmol) Kaliumhydroxid in 30 ml Methanol versetzt. Man erhitzt 10 min auf Rückflußtemperatur und läßt abkühlen. Die erhaltene Lösung wird mit 6 g Kieselgel versetzt und vom Lösungsmittel befreit. Zur Abtrennung von anorganischen Salzen wird das so erhaltene Pulver auf eine kurze Kieselgelsäule aufgegeben und mit Methyl-*tert*.-butylether/Petrolether = 1 : 2 v/v eluiert. Man erhält nach Verdampfen des Lösungsmittels 822 mg (2.92 mmol, 93%) Rohprodukt, welches aus wenig Methanol umkristallisiert wird. Es fallen 491 mg (1.75 mmol, 56%) N-Formyl-Dichlormethylen-TaClo als farblose Kristalle aus, die sich oberhalb 187°C zersetzen. Durch Einengen der Mutterlauge läßt sich weiteres Produkt isolieren, welches geringfügig mit der N-deformylierten Verbindung verunreinigt ist.
5.2 Zur Synthese schwächer (z.B. nur zweifach) halogenierter Vertreter läßt sich die CCl₃-Gruppe leicht mit verschiedenen Reduktionsmitteln reduzieren, z.B. mit Natriumborhydrid in absolutem Methanol oder mit Wasserstoff und Pd/C als Katalysator in 80proz. Methanol. Altemativ kann die Substanz auch *de novo,* z.B. aus Dichloracetaldehyd und den entsprechenden Tryptamin-Derivaten gewonnen werden: 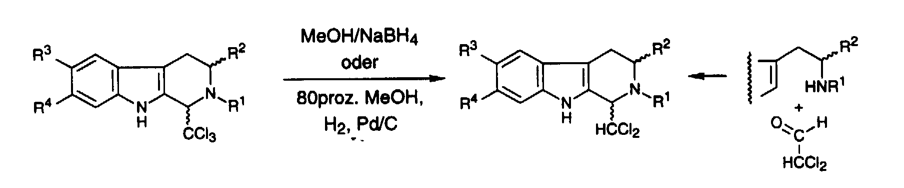 2.0 g (6.13 mmol) TaClo (Hydrochlorid) werden in 60 ml Methanol gelöst und unter Eiskühlung in mehreren Portionen mit insgesamt 10 g (0.26 mol) Natriumborhydrid versetzt. Es entsteht ein dicker Brei, der mit 30 ml Methanol versetzt wird und noch 4 h bei Raumtemperatur gerührt wird. Nach Zugabe von 10 g Kieselgel wird vom Lösungsmittel befreit, und das erhaltene Pulver wird auf eine Kieselgel-Säule (Laufmittel: Methyl-*tert*.-butylether/Petrolether = 1 : 2 v/v) aufgegeben und chromatographiert. Das Eluat wird mit methanolischer HCI versetzt, vom Lösungsmittel befreit und aus Methanol/Methyl-*tert*.-butylether umkristallisiert. Man erhält 402 mg (1.38 mmol, 22.5%) 1-Dichlormethyl-TaClo (Hydrochlorid).
   Ähnlich können auch Analoga mit längerkettigen Substituenten an C-1 z.B. aus halogenierten Aldehyden gewonnen werden: 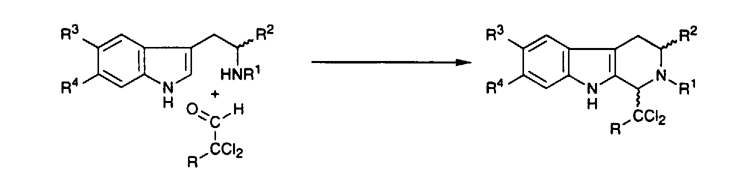
5.3 Zu Analoga mit anderen Halogenen, z.B. den ganz neuen 1-Tribrommethylverbindungen gelangt man durch Pictet-Spengler-Reaktion der Amine mit Bromal in Dioxan, Toluol, Dichlormethan und Aceton. Die Aufarbeitung ist jedoch relativ schwierig, so daß besser die gut kristallisierende *N*-Formylverbindung hergestellt und isoliert wird. Dazu wird das Amin mit Bromal in Ameisensäure bei Raumtemperatur gerührt. Es entstehen dabei zu etwa gleichen Teilen die *N*-Formyl- und die NH-Verbindung. Durch Zugabe von Formylierungsreagenz kann letztere nachformyliert werden, so daß einheitlich N-acyliertes Produkt vorliegt. Weitere Umsetzungen sind analog zu den 1-Trichlormethyl-β-carbolinen möglich: 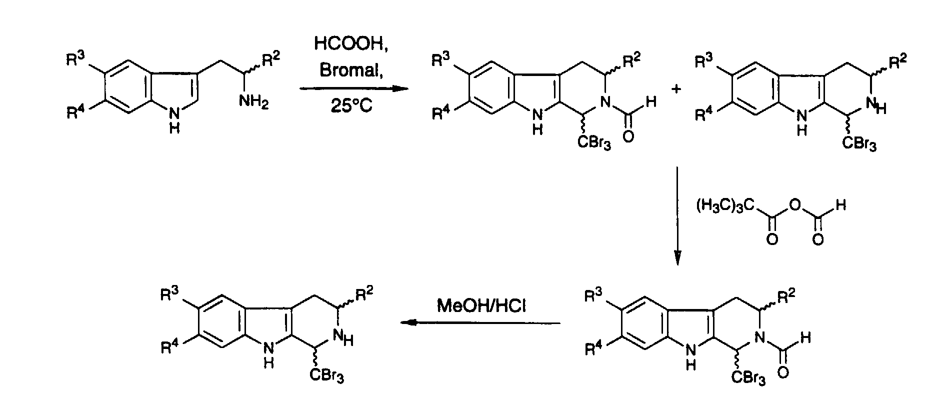 Weitere Herstellungs- und Umwandlungsmöglichkeiten der CBr₃-Verbindungen folgen den o.g. Verfahren zur Synthese der CCl₃-Analoga.
   5.0 g (30.9 mmol) Tryptamin, 17.4 g (61.8 mmol) Bromal und 7 ml Ameisensäure werden 5 h bei Raumtemp. gerührt und anschließend mit 4.41 g (33.9 mmol) Formylpivaloylanhydrid versetzt. Nach weiteren 6 h Rühren bei Raumtemp. wird die Reaktionslösung mit Eiswasser hydrolysiert, der ausgefallene Feststoff abfiltriert und mit 20 ml Methyl*tert*.-butylether gewaschen. Man erhält 7.05 g (15.7 mmol, 51%) der *N*-formylierten Verbindung (TaBro) als farbloses Kristallpulver mit Zersetzungspunkt 172°C.
   Zur Deformylierung werden 1.0 g (2.23 mmol) der *N*-formylierten Verbindung in einer Lösung von 2.8 ml konzentrierter Salzsäure in 20 ml Methanol suspendiert und 7 h unter Rückfluß erhitzt. Anschließend wird die Reaktionslösung im Vakuum auf 1/5 eingeengt und der ausgefallene Feststoff abfiltriert. Man erhält 0.92 g (2.00 mmol, 90% d.Th.) TaBro in Form seines Hydrochlorids als farbloses Kristallpulver. Schmp.: 162°C (Zers.).
   Analoge Umsetzungen betreffen auch die chemisch deutlich stabileren Derivate mit einer 1-Trifluormethylgruppe. 1-Trifluormethyl-3,4-didehydro-β-carbolin läßt sich gut nach den bei den 1-Trichlormethylderivaten geschilderten Methoden darstellen, z.B. in einer Bischler-Napieralski-Reaktion (vgl. 2.1): 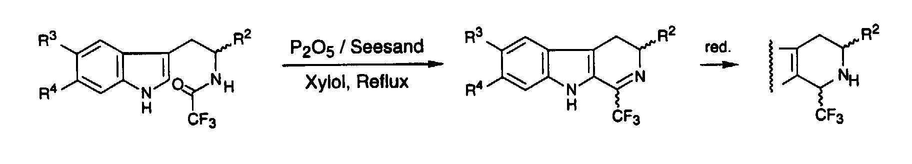 510 mg (2.00 mmol) Trifluoracetyltryptamid werden in 50 ml wasserfreiem Xylol bei 110°C innerhalb von 3 - 45 min in mehreren Portionen mit einem Gemisch aus 5 g Phosphorpentoxid und 10 g Seesand versetzt. Die Dehydratisierung erfolgt bei 6stdg. Erhitzen auf Rückflußtemperatur. Nach Abkühlen auf Raumtemperatur wird abfiltriert und der Rückstand in 200 g Eis/Wasser aufgeschlämmt. Unter Eiskühlung versetzt man die saure Lösung mit NaOH bis pH 8 - 9, extrahiert erschöpfend mit Dichlormethan und trocknet die vereinigten organischen Phasen über Natriumsulfat. Nach Einengen im Vakuum wird das so erhaltene Rohprodukt an Kieselgel (Laufmittel: Ether) chromatographiert. Man erhält 180 mg (0.76 mmol, 38%) leicht gelb gefärbte Kristalle vom Schmp. 84°C.
   Auch hier sind, wie oben für die CCl₃-Verbindungen beschrieben, nachträgliche Derivatisierungs- und Umwandlungsreaktionen möglich.

### Beispiel 6

### Synthese von enantiomerenreinem Material

Chirale Strukturen und damit verschiedene Enantiomere treten bei der Grundstruktur (**1**) für R² = H und bei Grundstruktur (**2**) für R² ≠ H auf.

Wegen der bekannten z.T. extrem unterschiedlichen Aktivität von Enantiomeren (Bild/Spiegelbild) in biologischen Systemen (vgl. den Fall Contergan) werden hier Methoden zur Reingewinnung stereochemisch einheitlicher Wirkstoffe vorgestellt:

### Darstellung enantiomerenreiner Verbindungen mit der Grundstruktur (1), R² = H:

6.1. Enantiomerenreines Material z.B. mit der Grundstruktur (**1**) läßt sich erhalten, indem man die *N*-Formylverbindungen (R¹ = CHO) langsam aus Methanol oder Aceton kristallisiert. Dabei können enantiomerenreine Kristalle erhalten werden, die sich anschließend ohne Verlust an optischer Reinheit *N*-deformylieren lassen. Der freie Stickstoff läßt sich dann wie oben beschrieben derivatisieren; die Zuordnung der absoluten Konfiguration erfolgt über CD-Spektroskopie oder Kristallstrukturanalyse.
   Eine kaltgesättigte Lösung von *N*-Formyl-TaClo in Aceton wird sorgfältig in ein offenes Kristallisiergefäß filtriert und bei Raumtemp. unter Lichtausschluß stehengelassen. Beim Verdunsten des Lösungsmittels wachsen langsam lange Kristallnadeln. Wenn diese etwa einen Zentimeter lang sind und zwischen 20 und 50 mg wiegen, bricht man die Kristallisation ab. Die Überprüfung der Enantiomerenreinheit einzelner Kristalle gelingt mittels HPLC an chiraler Phase (Chiralcel OD, Daicel Chemical Industries, LTD.; Petrolether/Isopropanol = 70 : 30 v/v; 1 ml/min). Das dabei schneller eluierende Enantiomer hat *S*-Konfiguration, das später eluierende ist entsprechend *R*-konfiguriert. Die Drehwerte des *S*- bzw. *R*-Enantiomeren sind [a]_{D}²⁵ = +87.5° (c = 0.481) bzw. [a]_{D}²⁵ = -87.3° (c = 0.393). 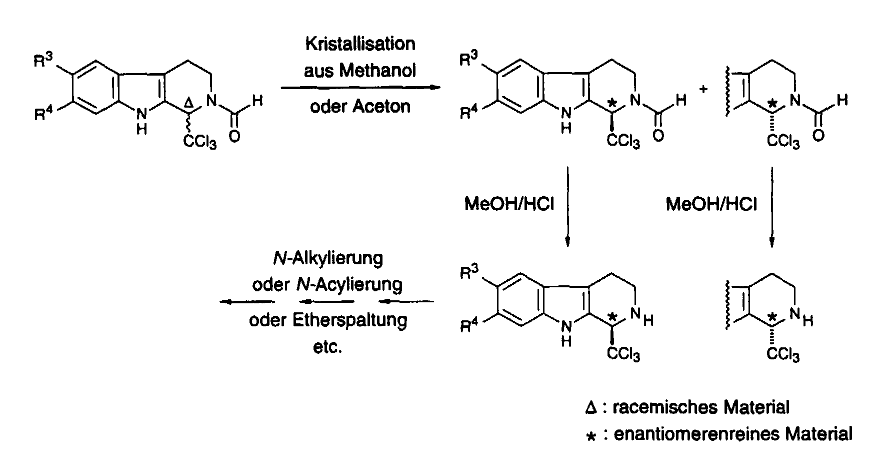 Nachfolgende Umsetzungen (Deformylierung, *N*-Methylierung usw.) können wie beim racemischen Material beschrieben durchgeführt werden.
6.2 Eine weitere Möglichkeit zur Enantiomerentrennung bietet die Chromatographie an chiraler Phase. Z.B. gelingt die Trennung in die Enantiomerenpaare bei den abgebildeten Substanzen problemlos z.B. durch HPLC mit einer Chiralcel-OD-Phase (Daicel Chemical Industries, LTD.) mit dem Fließmittel Petrolether/Isopropanol (70:30, v/v), aber auch andere Derivate lassen sich auf diese Weise trennen. *N*-Formyl-TaClo, N-Me-TaClo und TaClo (R³ = R⁴ = H) lassen sich per HPLC mit den o.g. Bedingungen sehr einfach trennen. Bei diesen drei Verbindungen besitzt jeweils das schneller eluierende Enantiomer *S*- und das langsamer eluierende *R*-Konfiguration: 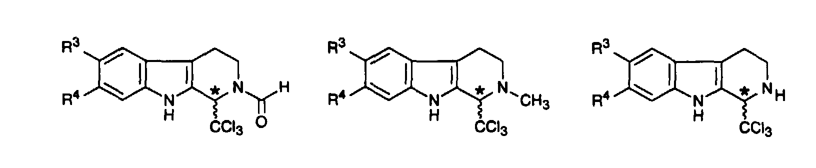
6.3 Auch die Erzeugung von Diastereomeren durch Salzbildung oder durch Derivatisierung am Stickstoff mit chiralen Hilfsreagenzien wie z.B. *R*-1-Phenylethylisocyanat, die nach der Trennung wieder abgespalten werden, bietet einen Zugang zu enantiomerenreinem Material: 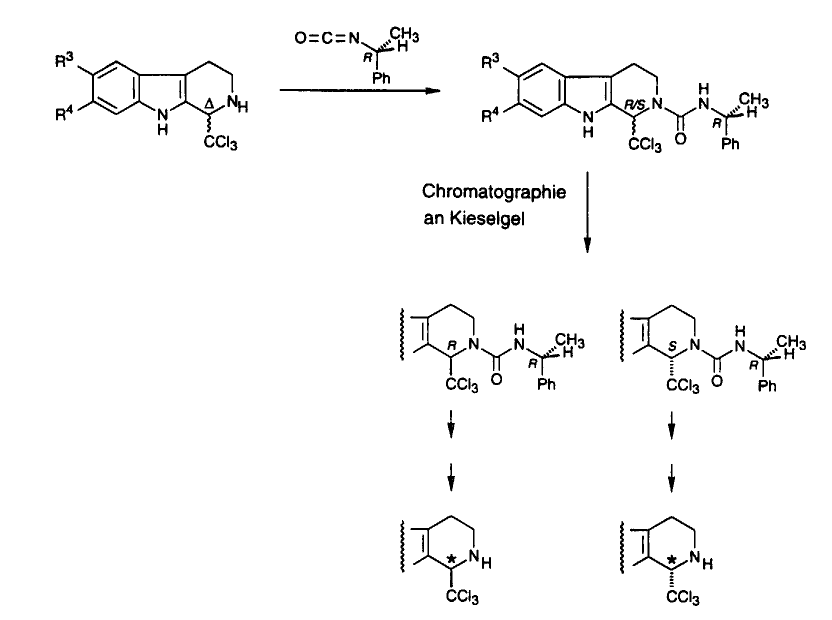 900 mg (3.11 mmol) TaClo (freie Base) und 4.80 ml (3.42 mmol) (*R*)-α-Phenylethylisocyanat werden zusammen mit katalytischen Mengen 4-Dimethylaminopyridin in 25 ml wasserfreiem Chloroform 4 h unter Rückfluß zum Sieden erhitzt. Nach Enffernen des Lösungsmittels wird der schwarze ölige Rückstand säulenchromatographisch an Kieselgel gereinigt (Laufmittel: Dichlormethan). Man erhält nach Enffernen des Lösungsmittels 886 mg (2.03 mmol, 65%) *N*-[(*R*)-1-Phenylethylcarbamoyl]-1-trichlormethyl-1,2,3,4-tetrahydro-β-carbolin in Form eines weißen Pulvers. Die so erhaltenen Diastereomere lassen sich z.B. durch HPLC oder durch Umkristallisation aus Chloroform/Petrolether oder Dichlormethan/Petrolether trennen. Durch zweimalige Umkristallisation von 100 mg des Diastereomerengemisches enthält man z.B. 31 mg eines reinen Diastereomers in Form farbloser Nadeln, die sich ab 178°C zersetzen.
6.4 Solche gut trennbaren Stereoisomere können auch durch Zugriff auf den "chiral pool", z.B. ausgehend von leicht verfügbaren enantiomerenreinen Tryptophanester-Derivaten erzeugt werden. Die Abspaltung der Esterfunktion liefert dann die enantiomerenreinen Derivate mit der Grundstruktur (**1**). Hier geht man von den kostengünstigen enantiomerenreinen L-Tryptophan-Derivaten aus. So kann die Trennung eines Enantiomerengemisches umgangen werden. Zur Synthese des anderen Enantiomeren bietet sich der Einsatz der ebenfalls kommerziell erhältlichen Derivate aus der D-Reihe an: 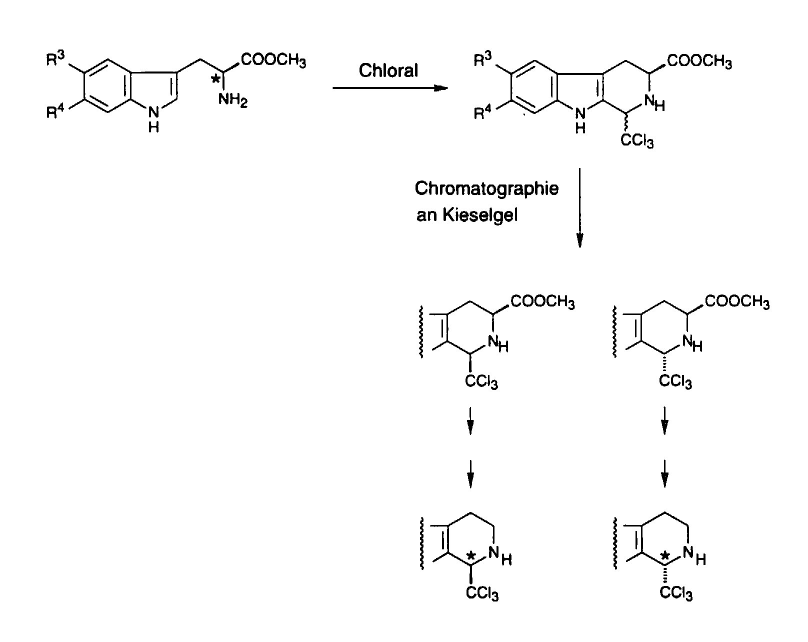 Darstellung enantiomerenreiner Verbindungen mit der Grundstruktur (**2**) bzw. (**3**), R² ≠ H:
6.5 Die "Hilfschiralität" des Tryptophanderivates kann natürlich auch im Molekül verbleiben und so z.B. zur Synthese enantiomerenreiner 3,4-Dihydro-β-carboline und 3,4-Dihydro-β-carboliniumsalze dienen. Die Abbildung zeigt ein typisches Beispiel:

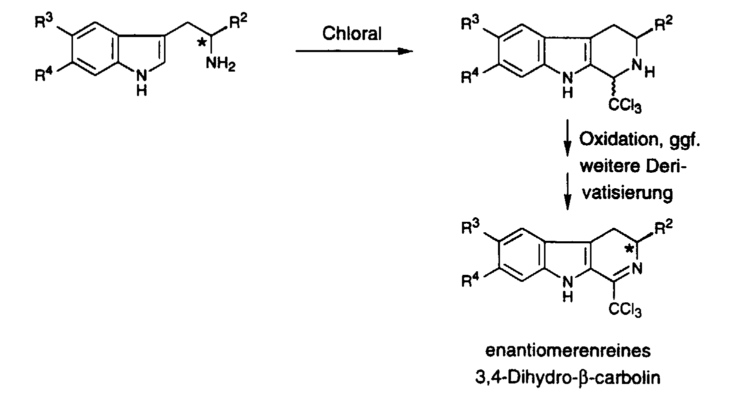

### Beispiel 7

### Stereoselektive gezielte Darstellung von diastereomerenreinem Material

Diastereomere treten auf bei der Grundstruktur (**1**) für R² ≠ H.
7.1 Zur Synthese werden die enantiomerenreinen Tryptophanderivate eingesetzt. Man erhält ein Paar enantiomerenreiner Diastereomere, welches sich durch Säulenchromatographie an Kieselgel mit Dichlormethan als Fließmittel trennen läßt (siehe oben).
   Ausgehend von enantiomeren reinem *N*_{b}-Benzyltryptophanmethylester erhält man bei der Kondensation mit Chloral in Toluol unter Rückfluß in Gegenwart katalytischer Mengen Trifluoressigsäure in hoher Stereoselektivität ausschließlich die *trans*-Diastereomeren. Nach Abspaltung der Benzylgruppe kann der freie Stickstoff nach den oben beschriebenen Methoden derivatisiert werden:

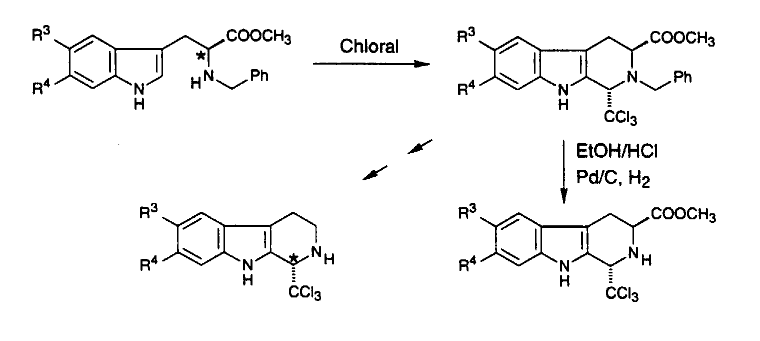

### Beispiel 8

### Einsatz der β-Carboline als Hemmstoffe insbesondere für Komplex I der Atmungskette; Spektralphotometrische Messung der Enzymaktivitäten an SMP's ("submitochondrial particles")

Die Isolierung von SMP's aus Rattenleber erfolgte nach (Ragan et al., 1987; Rickwood et al., 1987).
8.1 Hemmung des Komplexes I der Atmungskette in SMP's durch *N*-Me-TaClo
   Komplex I (NADH-Dehydrogenase) der mitochondrialen Atmungskette katalysiert im ersten Schritt eine NADH-Oxidation. Die dabei freiwerdenden Elektronen werden in einem zweiten Schritt auf Coenzym Q bzw. Ubichinon übertragen. Ein typischer Testansatz (modifiziert nach Ragan et al., 1987) enthält dabei 20 mM K-Phosphat Puffer, pH 8.0, 0.2 mM NADH, 1 mM KCN, SMP (ca. 0.3 mg Protein/ml) und +/- 250 µM *N*-Me-TaClo (25 mM methanolische Stammlösung). Der Start der Reaktion erfolgt durch Zugabe von 50 µM Ubichinon-1. Die Messung der Reaktion erfolgt spektralphotometrisch bei einer Wellenlänge von 340 nm während der linearen Phase. Bei Zugabe von *N*-Me-TaClo erfolgt eine 100proz. Hemmung der Ubichinon-Reduktion (s.a. Tabelle 1).
8.2 Hemmung der elektronenübertragenden Reaktionen auf Coenzym Q: NADH-Cytochrom-c-Reduktase und Succinat-Cytochrom-c-Reduktase
   Coenzym Q wird sowohl durch freiwerdende Elektronen bei der Komplex-I-katalysierten NADH-Oxidation, als auch bei der Komplex-II (Succinat-Coenzym-Q-Reduktase) -katalysierten Reaktion reduziert. Messungen dieser Reaktionswege mit anschließendem Elektronenfluß über den Komplex III bis zur Cytochrom-c-Reduktion lassen sich mittels folgender Tests untersuchen und durch die hier vorgestellten halogenierten β-Carbolinderivate (im folgenden am Beispiel von *N*-Me-TaClo) hemmen:
   - NADH-Cytochrom-c-Reduktase-Test (modifiziert nach Sottocasa et al., 1967)
   Ein typischer Testansatz enthält 25 mM K-Phosphat-Puffer, pH 7.5, 0.2 mM NADH, 0.1 mM Cytochrom-c, 0.5 mM KCN und +/- 250 µM N-Me-TaClo (25 mM methanolische Stammlösung). Die Messung der Reaktion erfolgt spektralphotometrisch bei einer Wellenlänge von 550 nm während der linearen Phase. Bei Zugabe von *N*-Me-Taclo erfolgt eine 100proz. Hemmung der Reaktion (s.a. Tabelle 1).
- Succinat-Cytochrom-c-Reduktase-Test (modifiziert nach Sottocasa et al., 1967)
   Ein typischer Testansatz enthält 55 mM K-Phosphat-Puffer, pH 7.5, 3 mM Na-Succinat, 0.1 mM Cytochrom c, +/- 250 µM N-Me-TaClo (25 mM methanolische Stammlösung) und 0.5 mM KCN. Gestartet wird die Reaktion mit SMP (ca. 0.3 mg Protein/ml). Die Messung der Reaktion erfolgt spektralphotometrisch bei einer Wellenlänge von 550 nm während der linearen Phase. Bei Zugabe von *N*-Me-TaClo erfolgt eine 100proz. Hemmung der Reaktion (s. auch Tabelle 1).
- Einsatz der halogenierten β-Carboline als Hemmstoffe der NADH- bzw. Succinat-gekoppelten Oxidation; Polarographische Messungen der Enzymaktivitäten an SMP's ("submitochondrial particles")
   Bei der polarographischen Messung wird die Aktivität der Atmungskette in ihrer Gesamtheit ausgehend von der NADH-Oxidation (Komplex I) bzw. Succinat-Oxidation (Komplex II) bis zur O₂-Reduktion (Komplex IV) bestimmt. Als Maß der Aktivität wird der O₂-Verbrauch bestimmt. Der Test wird unter Verwendung einer Clark-Sauerstoffelektrode (Rank Brothers, England) bei 30°C in 2 ml Puffer (50 mM K-Phosphat, pH 7.4, 250 mM Saccharose) und 0.15 mg SMP-Protein/ml durchgeführt. Nach 5 minütiger Vorinkubation ± Inhibitor wird die Messung mit NADH (Endkonzentration 0.28 mM) bzw. Succinat (Endkonzentration 20 mM) gestartet. Bei der Succinat-gekoppelten Oxidation werden die SMP's vorher nach Krueger et al., (1993) mit Malonat aktiviert.

**Tabelle 1:**

| Hemmung der mitochondrialen Atmungskette in Rattenhirn-Homogenaten am Beispiel von *N*-Me-TaClo: | |
|---|---|
| **Enzym** | **Inhibierung bei 250 µM *N*-Me-TaClo** |
| NADH-Oxidase | 0% |
| NADH-Ubichinon-1 -Reduktase | 100% |
| NADH-Cytochrom-c-Reduktase | 70% |
| Succinat-Cytochrom-c-Reduktase | 80% |
| Succinat-Dehydrogenase | 100% |

**Tabelle 2:**

| Hemmung der mitochondrialen Atmungskette in Rattenhirn-Homogenaten durch TaClo: | | | | |
|---|---|---|---|---|
| **Atmungsketten enzyme** | Enzymaktivität (nmol/min/mg Protein) | | | Inhibierungsrate durch TaClo |
| | Kontrolle | 500 µM TaClo | 50 µM Rotenon | |
| Komplex I | | | | |
| NADH-Oxidase | 141.0 | 138.0 | - | 0% |
| NADH CoQ1 Reduktase | 10.9 | 1.6 | 4.2 | 85% |

| Komplex II | | | | |
|---|---|---|---|---|
| Succinat Dehydrogenase | 29.6 | 30.1 | - | 0% |

| Komplex I/III | | | | |
|---|---|---|---|---|
| NADH Cyt c Reduktase | 24.6 | 14.7 | 5.7 | 40% |

| Komplex II/III | | | | |
|---|---|---|---|---|
| Succinat Cyt c Reduktase | 11.9 | 9.2 | - | 22% |

| Komplex IV | | | | |
|---|---|---|---|---|
| Cytochrom c Oxidase | 45.8 | 38.9 | - | 20% |
| Cyt c, Cytochrom c; CoQ1, Coenzym Q1, Ubichinon-1 | | | | |

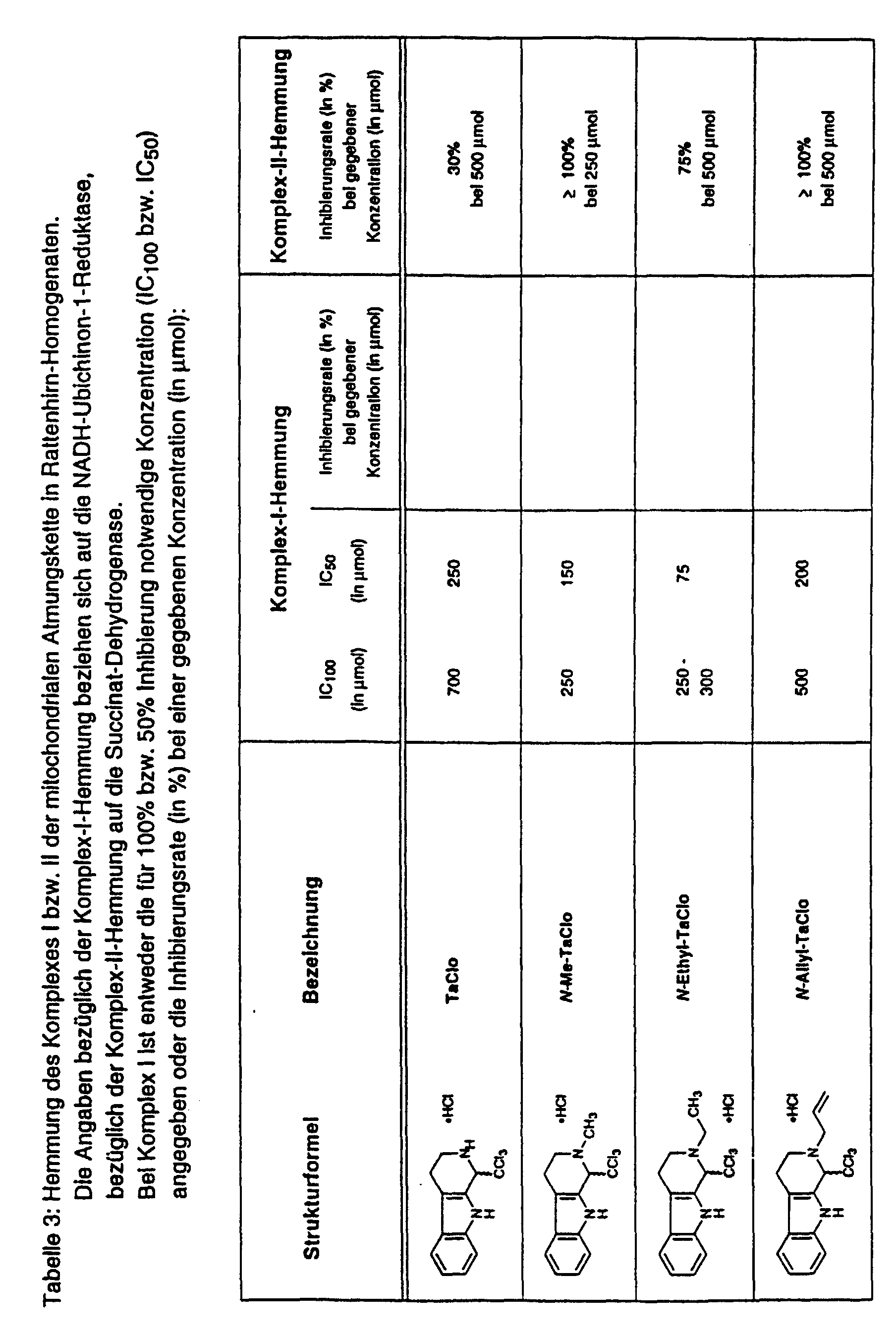

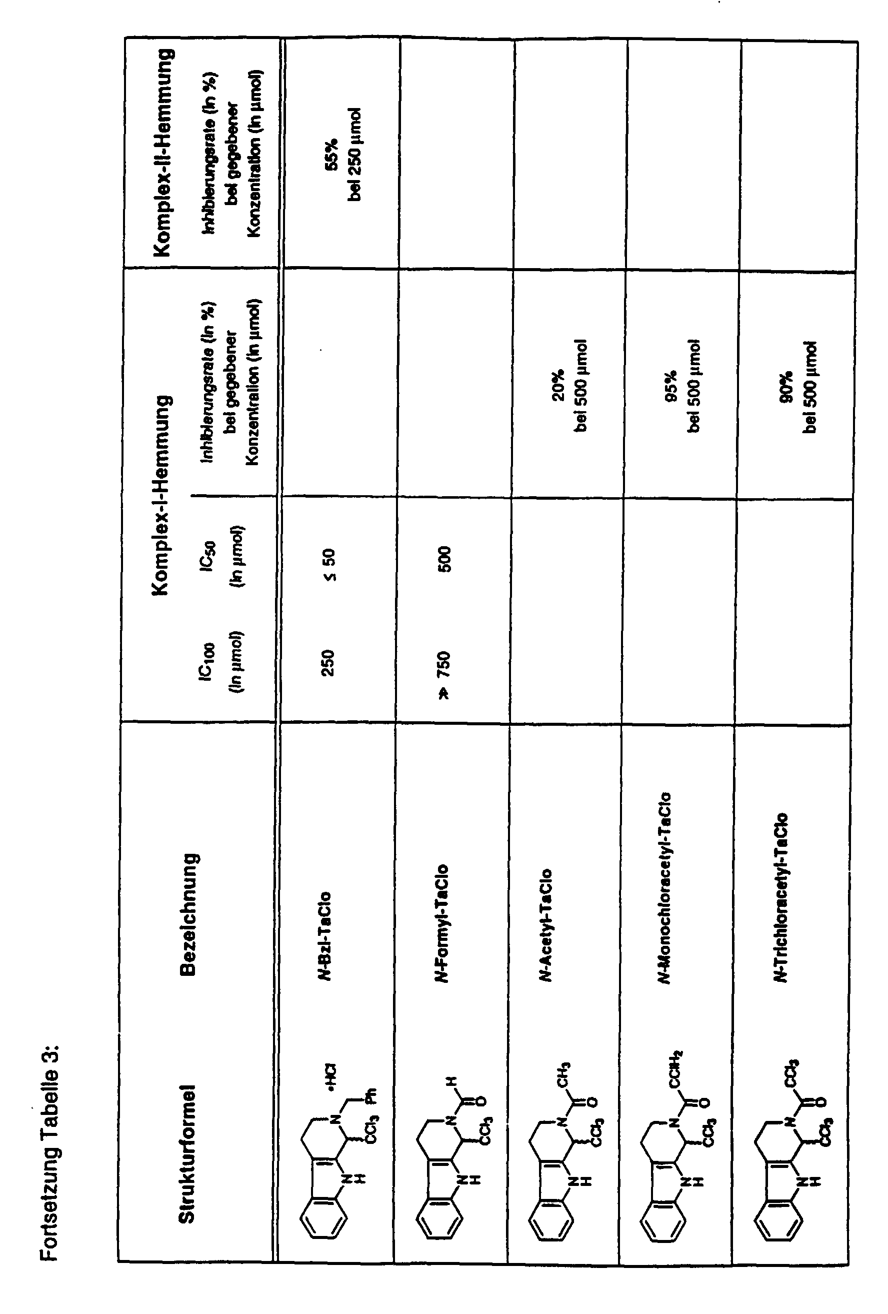

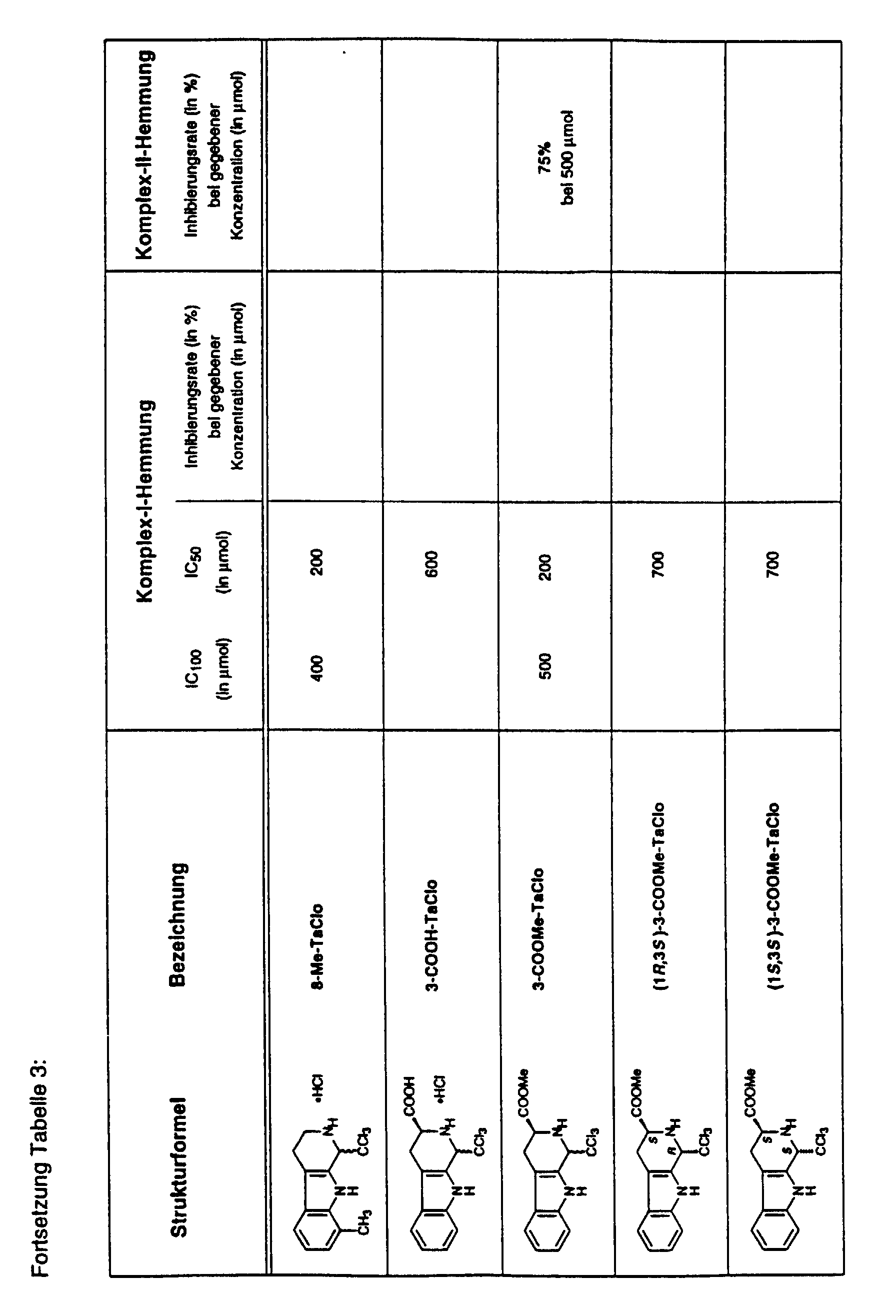

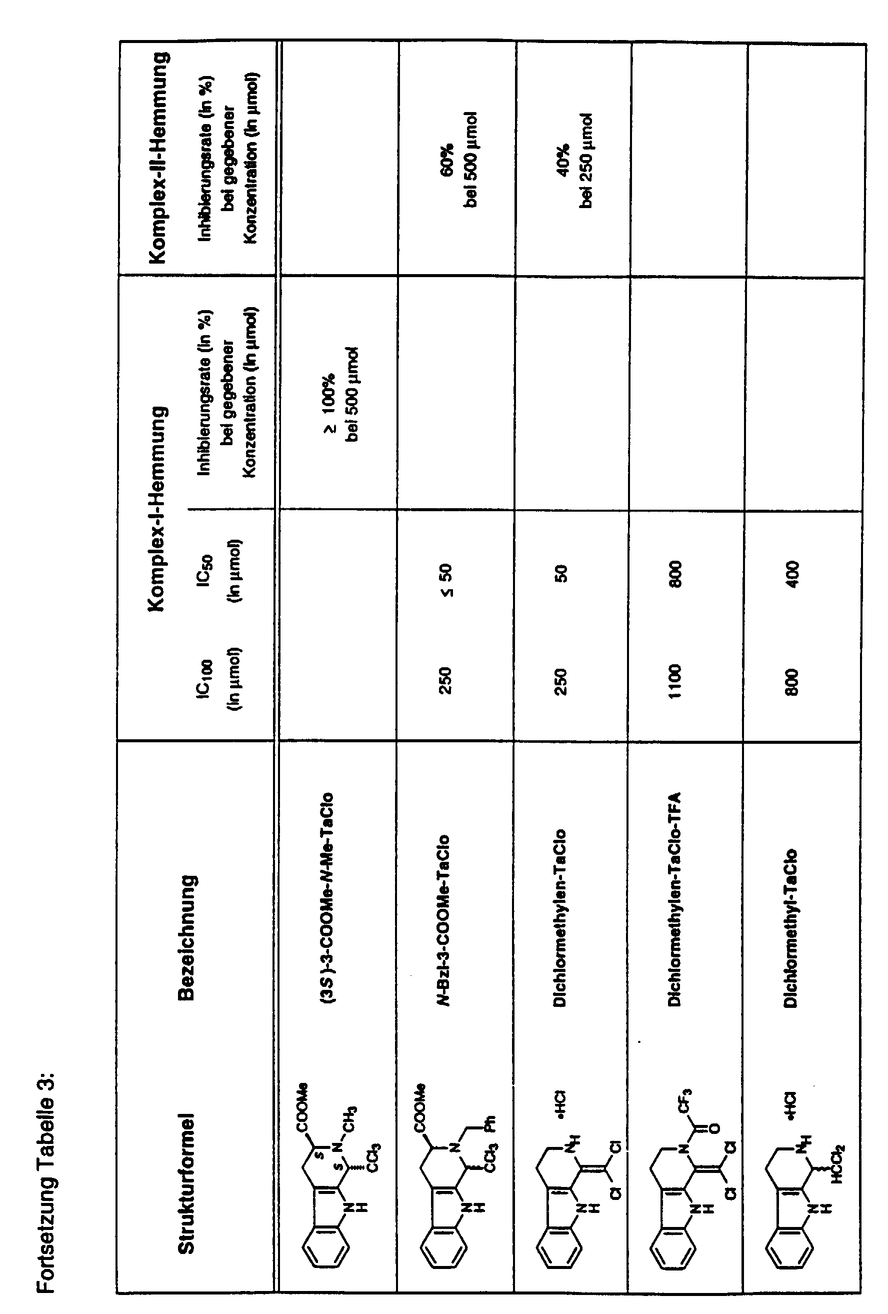

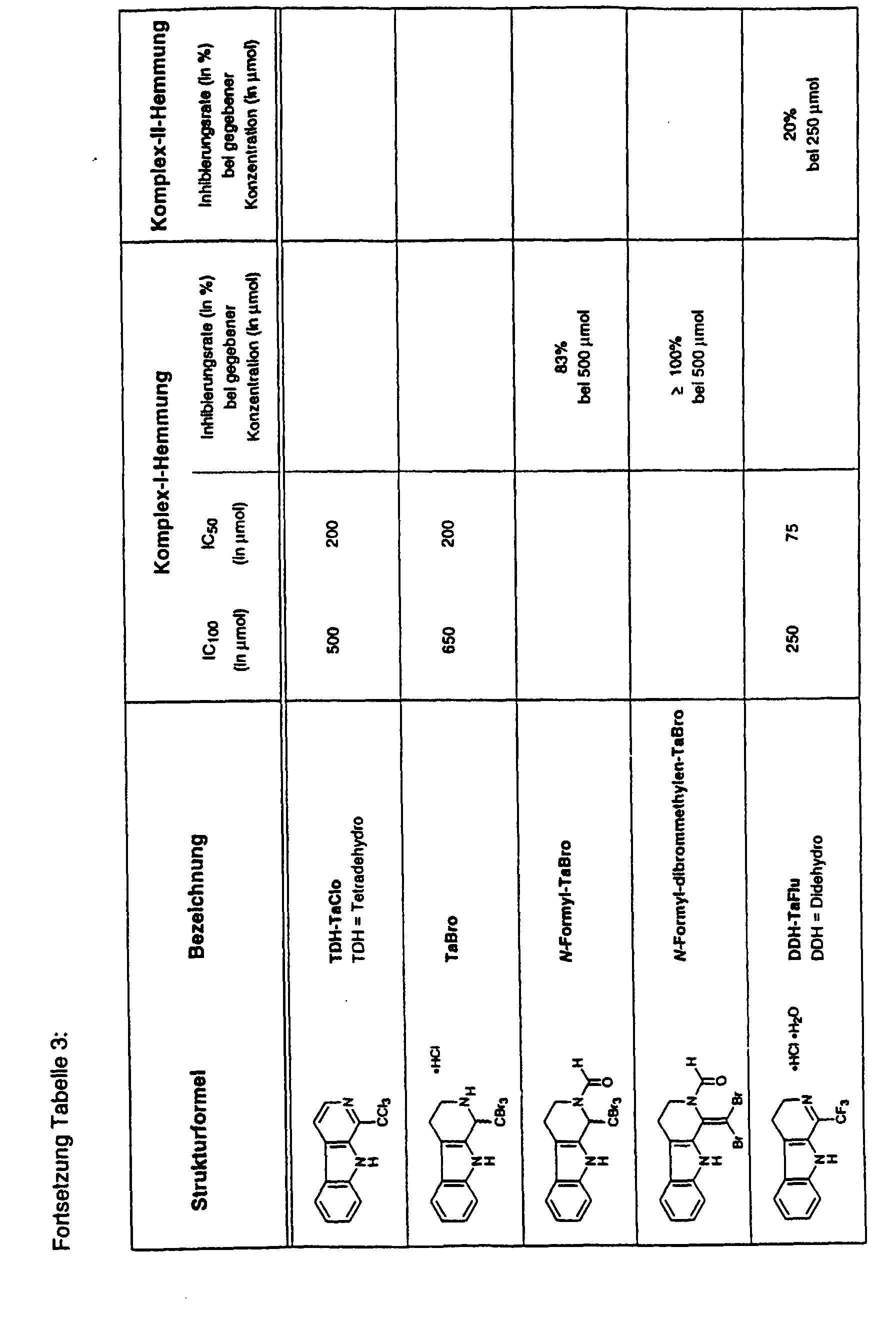

Nachfolgend werden noch bevorzugte Verbindungen der erfindungsgemäßen β-Carboline aufgezählt. Dies sind halogenierte β-Carboline der vorn erwähnten Formel (1) bzw. der Formeln (1) - (5), wobei R¹ = C₁-C₁₈Alkyl und X und R²-R⁴ die bei der Formel (I) angegebenen Bedeutungen haben. Weiter ist bevorzugt, wenn R² = -COOH, -COOCH₃, -CH₂OH, -COOC₂H₅, -COOCH₂-CH =CH₂, -COOCH₂-C₆H₅, -COONH₂, -CO₂NR₂, -CH₂OAc ist und X, R¹, R³, R⁴ die bei der Formel (I) angegebenen Bedeutungen haben.

Desweiteren sind halogenierte β-Carboline der Formel (I) bzw. der Formeln (1)-(5) bevorzugt, wobei R⁴ = H und R³ = H, HO-, CH₃O-, C₂H₅O-, C₆H₅-CH₂O-, CH₃COO-, C₂H₅COO- ist, oder wobei R³ = H und R⁴ = H, HO-, CH₃O-, C₂H₅O-, C₆H₅-CH₂O-, CH₃COO-, C₂H₅COO- ist und X, R¹, R² jeweils die bei der Formel (I) angegebenen Bedeutungen haben.

Weitere bevorzugte β-Carboline der Formel (I) bzw. der Formeln (1)-(5) sind solche, wobei X eine =C(Hal)₂-Gruppe mit Hal = F, Cl, Br, I ist und R¹- R⁴ die bei Formel (I) definierten Bedeutungen haben. Dabei ist =C(Hal)₂ bevorzugt = C(Cl)₂ oder = C(Br)₂.

Bei den β-Carbolinen der Formel (1) ist es bevorzugt, wenn die X-Gruppe eine -C(F)₃, -C(Cl)₃ oder -C(Br)₃-Gruppe ist, wobei R¹ - R⁴ die bei Formel (I) angegebenen Bedeutungen haben.

Allgemein sind auch β-Carboline der Formel (1) in enantiomerenreiner Form bevorzugt, wobei die X-Gruppe eine -C(Hal)₃-Gruppe mit Hal = F, Cl, Br, I ist und R²= H und R¹, R³, R⁴ haben die bei Formel (I) angegebenen Bedeutungen. Dabei kann die -C(Hal)₃-Gruppe eine -C(F)₃-Gruppe, -C(Cl)₃-Gruppe oder -C(Br)₃-Gruppe sein.

Bei Verbindungen der Formeln (1) - (3) ist es bevorzugt, wenn R² = -COOH, -COOCH₃, -COOC₂H₅, -COOCH₂-CH = CH₂, -COOCH₂-C₆H₅, -COONH₂, -CO₂NR₂, -CH₂OAc, -CH₂OH ist und X, R¹, R³, R⁴ die bei der Formel (I) definierten Bedeutungen haben und ganz bevorzugt in diastereomerenreiner Form gewonnen werden.

Für Verbindungen der Formel (1) ist es auch bevorzugt, wenn R¹ = -COCCl₃ bzw. -COCH₂Cl oder -COCHCl₂ ist und X, R²- R⁴ die bei Formel (I) angegebenen Bedeutungen haben.

Für Verbindungen der Grundstruktur (I) ist es auch bevorzugt, wenn R¹-R⁴ = H und X = C(Cl)₃ oder C(Br)₃ oder C(F)₃ ist.

Diese vorgenannten bevorzugten Verbindungen sowie die schon weiter vorn genannten Verbindungen eignen sich alle zur Herstellung einer Zusammensetzung zur Hemmung der Atmungskette.

### Literatur

Bringmann G, Hille A (1990) 1-Trichloromethyl-1,1,1,4-tetrahydro-β-carboline-A Potential Chloral-Derived Indol Alkaloid in Man. *Arch. Pharm.* 111:567-569.
Bringmann G, Hille A, Stäblein M, Peters P, von Schnering HG (1991) Potential Tryptophan-Derived Alkaloids in Chloral-Treated Patients: Synthesis and Stereostructure. *Liebigs Ann. Chem.* 1189-1194.
Ragan Cl, Wilson MT, Darley-Usmar VM, Lowe PN (1987) Sub-fractionation of mitochondria and isolation of the proteins of oxidative phosphorylation. In: *Mitochondria - a practical approach* (eds. Darley-Usmar VM, Rickwood D, Wilson MT), pp 79-111.
Rickwood D, Wilson MT, Darley-Usmar VM (1987) Isolation and characteristics of intact Mitochondria. In: *Mitochondria - a practical approach* (eds. Darley-Usmar VM, Rickwood D, Wilson MT), pp 1-16.
Sottocasa GL, Kuylenstierna B, Ernster L, Bergstrand A (1967). An electron transport system associated with the outer membrane of the mitochondria. J. *Cell Biol.* 11:415-418.

## Patentansprüche

1. Halogenierte β-Carboline mit der Formel (I) 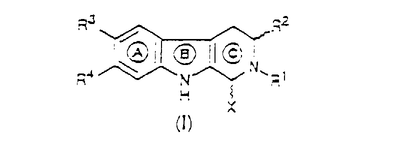 wobei in Ring C eine, zwei oder drei Doppelbindungen vorhanden sind,
X die Bedeutung -C(Hal)₃, -CH(Hal)₂, -CH₂(Hal) oder =C(Hal)₂ mit Hal = Cl, Br, I hat, und
die Substituenten R¹, R², R³ und R⁴ dabei folgende Bedeutungen haben:
R¹ : -H, eine C₁-C₁₈-Alkylgruppe (z.B. Methyl, Ethyl, Propyl, *n*-Butyl, *iso*-Butyl, *tert*.-Butyl usw.), -CH₂-CH=CH₂, -CH₂-C₆H₅, eine Acyl- oder Aroylgruppe, mit der Maßgabe, daß R¹ auch ein freies Elektronenpaar sein kann, wenn der Stickstoff an der Ausbildung einer Doppelbindung beteiligt ist.
R²: -H, -COOH, -COOCH₃, -COOC₂H₅, -COOCH₂-CH=CH₂, -COOCH₂-C₆H₅, -COONH₂, -CO₂NR₂, -CH₂OAc, -CH₂-OH.
R³: H-, HO-, Ch₃O-, C₂H₅O-, C₆H₅-CH₂O-, CH₃COO-, C₂H₅COO-,
R⁴: H-, HO-, CH₃O-, C₂H₅O-, C₆H₅-CH₂O-, CH₃COO-, C₂H₅COO-,
wobei der Stickstoff im Indolring ggf. alkyliert oder acyliert ist und wobei ggf. in in den Positionen 4 und/oder 5 und/oder 8 des β-Carbolingerüsts vorhandene Substituenten ausgewählt sind aus OH-, OCH₃-, CH₃-, F-, Cl-, Br-, I-,
mit der Maßgabe, daß die folgenden Verbindungen ausgenommen sind: 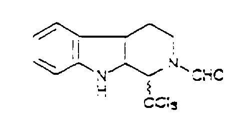 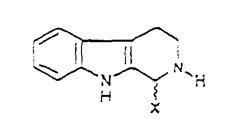 wobei X die Bedeutung -CCl₃ oder -CH₂Cl hat. 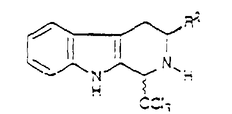 wobei R² die Bedeutung -COOH oder -COOCH₃ hat, 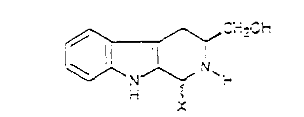 wobei X die Bedeutung -CCl₃ oder CHCl₂ hat, 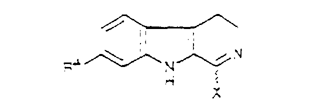 wobei X und R⁴ die folgenden Bedeutungen haben:
X: -CCl₃ und R⁴:H-,
X: -CH₂Cl und R⁴: H-,
X: -CHBr₂ und R⁴: CH₃O-,
X: -CH₂Br und R⁴: CH₃O-.

2. Halogenierte β-Carboline nach Anspruch 1, wobei diese eine der folgenden Formeln (**1**)-(**5**) erfüllen: 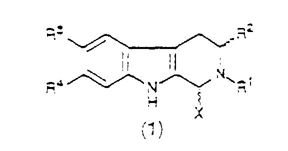 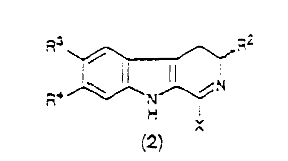 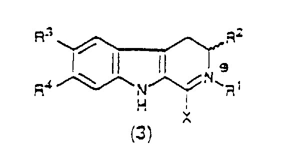 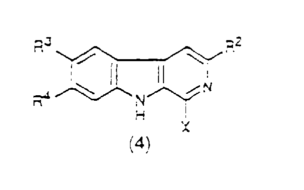 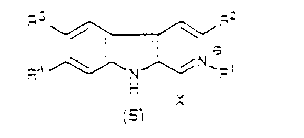 und X, R¹-R⁴ die in Anspruch 1 definierten Bedeutungen haben.

3. Halogenierte β-Carboline nach Anspruch 1 oder 2, wobei X eine = C(Hal)₂-Gruppe mit Hal = Cl, Br, I ist und R¹-R⁴ die in Anspruch 1 definierten Bedeutungen haben.

4. Halogenierte β-Carboline nach Anspruch 2, wobei in Formel (1) die X-Gruppe eine -C(Hal)₃-Gruppe mit Hal = Cl, Br, I ist und R² = H und R¹, R³, R⁴ die in Anspruch 1 definierten Bedeutungen haben und die Verbindungen enantiomerenrein sind.

5. Verwendung von β-Carbolinen mit der Formel (I) 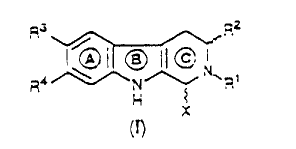 wobei in Ring C eine, zwei oder drei Doppelbindungen vorhanden sind,
X die Bedeutung -C(Hal)₃, -CH(Hal)₂, -CH₂(Hal) oder =C(Hal)₂ mit
Hal = Fn, Cl, Br, I hat, und
die Substituenten R¹, R², R³ und R⁴ dabei folgende Bedeutungen haben:
R¹ : -H, eine C₁-C₁₈-Alkylgruppe (z.B. Methyl, Ethyl, Propyl, *n*-Butyl, *iso*-Butyl, *tert*.-Butyl usw.), -CH₂-CH=CH₂, -CH₂-C₆H₅, eine Acyl- oder Aroylgruppe, mit der Maßgabe, daß R¹ auch ein freies Elektronenpaar sein kann, wenn der Stickstoff an der Ausbildung einer Doppelbindung beteiligt ist.
R²: -H, -COOH, -COOCH₃, -COOC₂H₅, -COOCH₂-CH=CH₂, -COOCH₂-C₆H₅, -COONH₂, -CO₂NR₂, -CH₂OAc, -CH₂-OH,
R³: H-, HO-, CH₃O-, C₂H₅O-, C₆H₅-CH₂O-, CH₃COO-, C₂H₅COO-
R⁴: H-, HO-, CH₃O-, C₂H₅O-, C₆H₅-CH₂O-, CH₃COO-, C₂H₅COO-,
zur Herstellung einer Zusammensetzung zur Hemmung der Atmungskette.

6. Verwendung nach Anspruch 5, wobei der Komplex I und/oder II der Atmungskette gehemmt wird.

7. Verwendung nach Anspruch 5 oder 6, wobei das β-Carbolin eine der folgenden Formeln (1)-(5) erfüllt 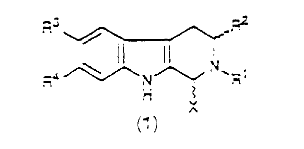 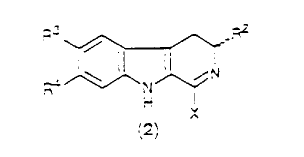 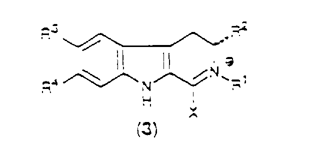 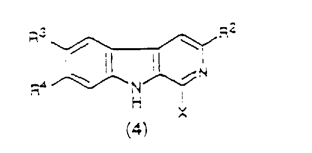 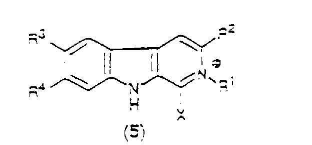 und X, R¹-R⁴ die in Anspruch 5 definierten Bedeutungen haben.

8. Verwendung nach einem der Ansprüche 5-7, wobei in Formel (I) R¹-R⁴ = H und X = C(Cl)₃ oder C(Br)₃ oder C(F)₃ ist.

9. Verwendung nach einem der Ansprüche 5-8, wobei X eine =C(Hal)₂-Gruppe mit Hal = F, Cl, Br, I ist und R¹-R⁴ die in Anspruch 5 definierten Bedeutungen haben.

10. Verwendung halogenierter β-Carboline nach Anspruch 7, wobei in Formel (1) die X-Gruppe eine -C(Cl)₃-Gruppe, -C(Br)₃-Gruppe oder -C(F)₃-Gruppe ist und R¹-R⁴ die in Anspruch 5 definierte Bedeutung haben.

11. Verwendung halogenierter β-Carboline in enantiomerenreiner Form nach Anspruch 7, wobei in Formel (1) die X-Gruppe eine -C(Hal)₃-Gruppe mit Hal = F, Cl, Br, I ist und R² = H und R¹, R³, R⁴ die in Anspruch 7 definierte Bedeutung haben.

12. Verwendung halogenierter β-Carboline nach Anspruch 7, wobei der Stickstoff im Indolring alkyliert oder acyliert ist und X, R¹-R⁴ die in Anspruch 5 definierten Bedeutungen haben.

13. Verwendung halogenierter β-Carboline nach einem der Ansprüche 5-12 mit OH-, OCH₃-, CH₃, F-, Cl-, Br- und I-Substituenten in den Positionen 4 und/oder 5 und/oder 8 des β-Carbolingerüsts, wobei X, R¹-R⁴ die in Anspruch 5 definierten Bedeutungen haben.

## Claims

1. Halogenated β-carbolines of the formula (1) 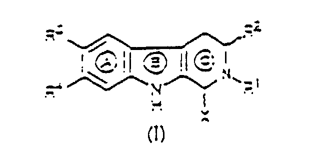 wherein there are one, two or three double bonds in the C ring,
X denotes -C(Hal)₃, -CH(Hal)₂, -CH₂(Hal) or =C(Hal)₂ with Hal = Cl, Br and I, and
the substituents R¹, R², R³ and R⁴ denote the following:
R¹: -H, a C₁-C₁₈-alkyl group (for example methyl, ethyl, propyl, n-butyl, iso-butyl, tert.-butyl etc), -CH₂-CH=CH₂, -CH₂-C₅H₅, an acyl or aroyl group, with the proviso that R¹ can also be a free electron pair if nitrogen is involved in the formation of a double bond,
R²: -H, -COOH, -COOCH₃, -COOC₂H₅, -COOCH₂-CH=CH₂, -COOCH₂- C₅H₅, -COONH₂, -CO₂NR₂, -CH₂OAc, -CH₂-OH,
R³: H-, HO-, CH₃O-, C₂H₅O-, C₅H₅-CH₂O-, CH₃COO-, C₂H₅COO-,
R⁴: H-, HO-, CH₃O-, C₂H₅O-, C₅H₅-CH₂O-, CH₃COO-, C₂H₅COO-,
wherein the nitrogen in the indole ring is possibly alkylated or acylated and wherein substituents possibly present in positions 4 and/or 5 and/or 8 of the β-carboline structure are selected from OH-, OCH₃-, CH₃-, F-, C1-, Br- and I-,
with the proviso that the following compounds are excluded: 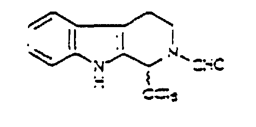 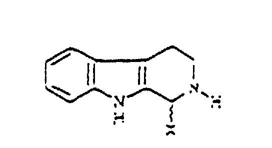 wherein X denotes -CCl₃ or -CH₂Cl, 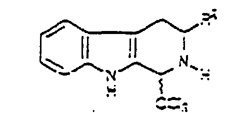 wherein R² denotes -COOH or -COOCH₃, 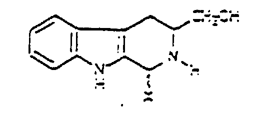 wherein X denotes -CCl₃ or CHCl₂, and 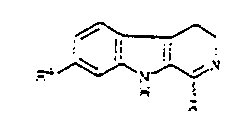 wherein X and R⁴ denote the following:
X: -CCl₃ and R⁴: H-,
X: -CH₂Cl and R⁴: H-,
X: -CHBr₂ and R⁴: CH₃O-,
X: -CH₂Br and R⁴: CH₃O-.

2. Halogenated β-carbolines according to claim 1 wherein they satisfy one of the following formulae (1)-(5): 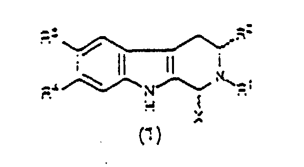 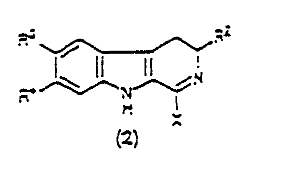 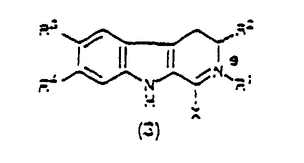 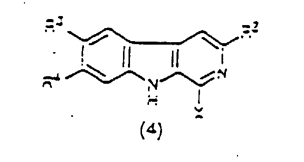 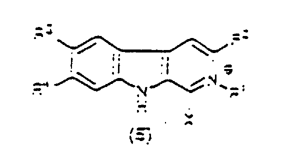 and X and R¹-R⁴ are as defined in claim 1.

3. Halogenated β-carbolines according to claim 1 or claim 2 wherein X is a =C(Hal)₂-group with Hal = Cl, Br and I and R¹-R⁴ are as defined in claim 1.

4. Halogenated β-carbolines according to claim 2 wherein in formulae (1) the X-group is a -C(Hal)₃-group with Hal = Cl, Br and I and R² = H and R¹, R³ and R⁴ are as defined in claim 1 and the compounds are enantiomer-free.

5. Use of β-carbolines of the formula (1) 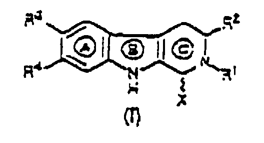 wherein there are one, two or three double bonds in the C ring,
X denotes -C(Hal)₃, -CH(Hal)₂, -CH₂(Hal) or -C(Hal)₂ with Hal = F, C1, Br and I, and
the substituents R¹, R², R³ and R⁴ denote the following:
R¹: -H, a C₁-C₁₈-alkyl group (for example methyl, ethyl, propyl, n-butyl, iso-butyl, tert.-butyl etc), -CH₂-CH=CH₂, -CH₂-C₅H₅, an acyl or aroyl group, with the proviso that R¹ can also be a free electron pair if nitrogen is involved in the formation of a double bond,
R²: -H, -COOH, -COOCH₃, -COOC₂H₅, -COOCH₂-CH=CH₂, -COOCH₂- C₅H₅, -COONH₂, -CO₂NR₂, -CH₂OAc, -CH₂-OH,
R³: H-, HO-, CH₃O-, C₂H₅O-, C₅H₅-CH₂O-, CH₃COO-, C₂H₅COO-,
R⁴: H-, HO-, CH₃O-, C₂H₅O-, C₅H₅-CH₂O-, CH₃COO-, C₂H₅COO-,
for the production of a composition for inhibition of the respiratory chain.

6. Use according to claim 5 wherein the complex I and/or II of the respiratory chain is inhibited.

7. Use according to claim 5 or claim 6 wherein the β -carboline satisfies one of the following formulae (1)-(5) 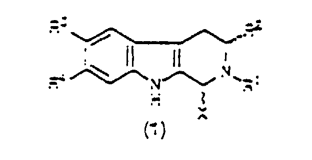 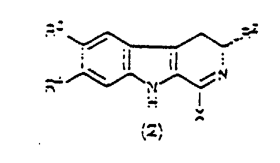 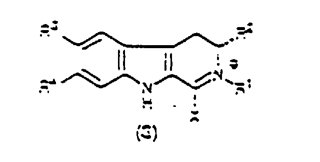 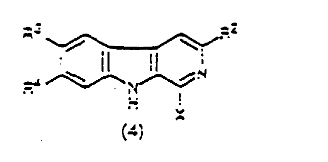 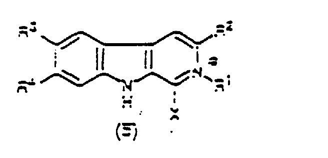 and X and R¹-R⁴ are as defined in claim 5.

8. Use according to one of claims 5 to 7 wherein in formula (1) R²-R⁴ = H and X = C(Cl)₃ or C(Br)₃ or C(F)₃.

9. Use according to one of claims 5 to 8 wherein X is a =C(Hal)₂-group with Hal = F, Cl, Br and I and R¹-R⁴ are as defined in claim 5.

10. Use of halogenated β-carbolines according to claim 7 wherein in formula (1) the X-group is a -C(Cl)₃-group, a -C(Br)₃-group or -C(F)₃-group and R¹-R⁴ are as defined in claim 5.

11. Use of halogenated β-carbolines in enantiomer-free form according to claim 7 wherein in formula (I) the X-group is a -C(Hal)₃-group with Hal = F, Cl, Br and I and R² = H and R¹, R³ and R⁴ are as defined in claim 7.

12. Use of halogenated β-carbolines according to claim 7 wherein the nitrogen in the indole ring is alkylated or acylated and X and R¹-R⁴ are as defined in claim 5.

13. Use of halogenated β-carbolines according to one of claims 5 to 12 with OH-, OCH₃-, CH₃, F-, C1-, Br- and I- substituents in positions 4 and/or 5 and/or 8 of the β-carboline structure, wherein X and R¹-R⁴ are as defined in claim 5.

## Revendications

1. β-carbolines halogénées de formule (I) 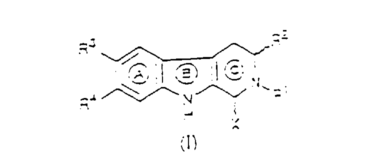 dans laquelle une, deux ou trois doubles liaisons sont présentes dans le noyau C,
X représente -C(Hal)₃, -CH(Hal)₂, -CH₂(Hal) ou =C(Hal)₂, où Hal = Cl, Br, I, et
les substituants R¹, R², R³ et R⁴ ont les définitions suivantes :
R¹ représente -H, un groupe alkyle en C₁ à C₁₈ (par exemple méthyle, éthyle, propyle, n-butyle, isobutyle, tertio-butyle, etc.), un groupe -CH₂-CH=CH₂, -CH₂-C₆H₅, un groupe acyle ou aroyle, sous réserve que R¹ puisse représenter aussi un doublet électronique libre lorsque l'azote participe à la formation d'une double liaison,
R² représente -H, un groupe -COOH, -COOCH₃, -COOC₂H₅, -COOCH₂-CH=CH₂, -COOCH₂-C₆H₅, -COONH₂, -CO₂NR₂, -CH₂OAc, -CH₂-OH, R³ : H-, HO-, CH₃O-, C₂H₅O-, C₆H₅-CH₂O-, CH₃COO-, C₂H₅COO-, R⁴: H-, HO-, CH₃O-, C₂H₅O-, C₆H₅-CH₂O-, CH₃COO-, C₂H₅COO-,
l'azote du noyau d'indole étant éventuellement alkylé ou acylé et des substituants éventuellement présents dans les positions 4 et/ou 5 et/ou 8 du sequelette de β-carboline étant choisi entre OH-, OCH₃-, CH₃-, F-, C1-, Br-, I-,
sous réserve que les composés suivants soient exclus : 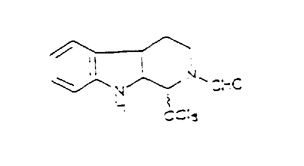 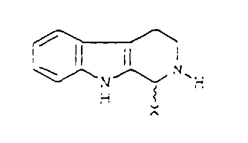 où X représente -CCl₃ ou -CH₂Cl, 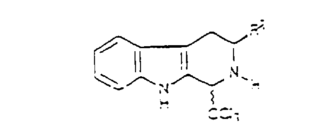 où R² représente -COOH ou -COOCH₃, 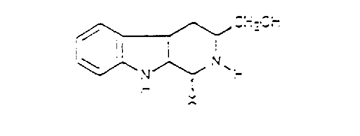 où X représente -CCl₃ ou CHCl₂, 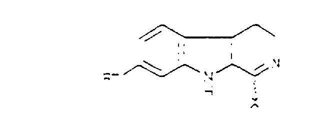 où X et R⁴ ont les définitions suivantes :
X : -CCl₃ et R⁴: H-,
X : -CH₂Cl et R⁴: H-,
X : -CHBr₂ et R⁴: CH₃O-,
X : -CH₂Br et R⁴ : CH₃O-.

2. β-carbolines halogénées suivant la revendication 1, qui satisfont à l'une des formules (1)-(5) suivantes :

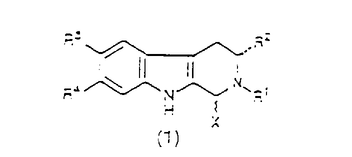

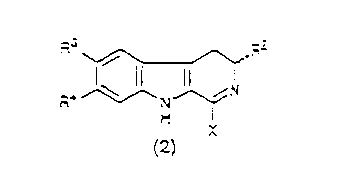

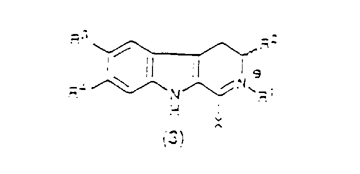

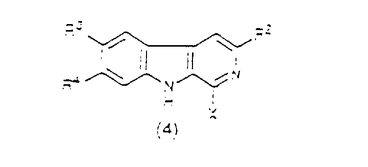

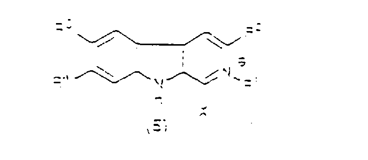

3. β-carbolines halogénées suivant la revendication 1 ou 2, dans lesquelles X est un groupe =C(Hal)₂, dans lequel Hal représente Cl, Br ou I et R¹-R⁴ ont les définitions indiquées dans la revendication 1.

4. β-carbolines halogénées suivant la revendication 2, dans la formule (1) desquelles le groupe X est un groupe -C(Hal)₃- dans lequel Hal représente Cl, Br ou I et R² représente H, et R¹, R³, R⁴ ont les définitions indiquées dans la revendication 1, et les composés sont des énantiomères purs.

5. Utilisation de β-carbolines de formule (I) 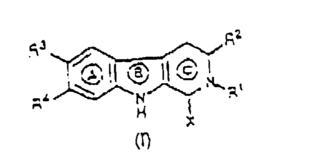 dans laquelle une, deux ou trois doubles liaisons sont présentes dans le noyau C,
X représente -C(Hal)₃, -CH(Hal)₂, -CH₂(Hal) ou =C(Hal)₂, où Hal = F, Cl, Br, I, et
les substituants R¹, R², R³ et R⁴ ont les définitions suivantes :
R¹ représente -H, un groupe alkyle en C₁ à C₁₈ (par exemple méthyle, éthyle, propyle, n-butyle, isobutyle, tertio-butyle, etc.), un groupe -CH₂-CH=CH₂, -CH₂-C₆H₅, un groupe acyle ou aroyle, sous réserve que R¹ puisse représenter aussi un doublet électronique libre lorsque l'azote participe à la formation d'une double liaison,
R² représente -H, un groupe -COOH, -COOCH₃, -COOC₂H₅, -COOCH₂-CH=CH₂, -COOCH₂-C₆H₅, -COONH₂, -CO₂NR₂, -CH₂OAc, -CH₂-OH, R³ : H-, HO-, CH₃O-, C₂H₅O-, C₆H₅-CH₂O-, CH₃COO-, C₂H₅COO-, R⁴: H-, HO-, CH₃O-, C₂H₅O-, C₆H₅-CH₂O-, CH₃COO-, C2H5COO-,
pour la préparation d'une composition destinée à inhiber la chaîne respiratoire.

6. Utilisation suivant la revendication 5, dans laquelle le complexe I et/ou II de la chaîne respiratoire est inhibé.

7. Utilisation suivant la revendication 5 ou 6, dans laquelle la β-carboline répond à l'une des formules (1)-(5) suivantes : 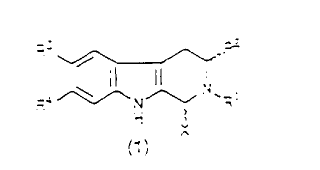 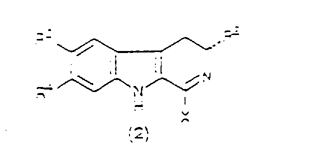 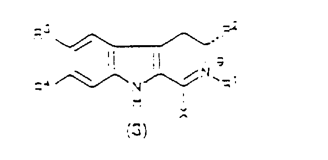 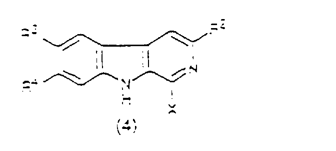 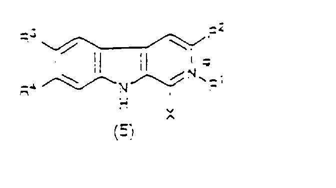 et X, R¹-R⁴ ont les définitions indiquées dans la revendication 5.

8. Utilisation suivant l'une des revendications 5 à 7, dans laquelle, dans la formule (I), R¹ à R⁴ représentent H et X est un groupe C(Cl)₃ ou C(Br)₃ ou C(F)₃.

9. Utilisation suivant l'une des revendications 5 à 8, dans laquelle X est un groupe =C(Hal)₂-, dans lequel Hal représente F, Cl, Br, I et R¹ à R⁴ ont les définitions indiquées dans la revendication 5.

10. Utilisation de β-carbolines halogénées suivant la revendication 7, dans laquelle, dans la formule (1), le groupe X est un groupe -C(Cl)₃-, un groupe -C(Br)₃-ou un groupe -C(F₃)- et R¹ à R⁴ ont les définitions indiquées dans la revendication 5.

11. Utilisation de β-carbolines halogénées sous la forme énantiomère pure selon la revendication 7, dans laquelle, dans la formule (1), le groupe X est un groupe -(Hal)₃- dans lequel Hal représente F, Cl, Br, I, et R² représente H et R¹, R³, R⁴ ont la définition indiquée dans la revendication 7.

12. Utilisation de β-carbolines halogénées suivant la revendication 7, dans laquelle l'azote du noyau d'indole est alkylé ou acylé et X, R¹ à R⁴ ont les définitions indiquées dans la revendication 5.

13. Utilisation de β-carbolines halogénées suivant l'une des revendications 5 à 12, avec les substituants OH-, OCH₃-, CH₃, F-, Cl-, Br- et I dans les positions 4 et/ou 5 et/ou 8 du squelette de β-carboline, X, R¹ à R⁴ ayant les définitions indiquées dans la revendication 5.
